# EUROPEAN PATENT SPECIFICATION

(11) **EP 3 866 920 B1**
(45) Date of publication and mention of the grant of the patent: **10.12.2025**
(21) Application number: 19787255.9
(22) Date of filing: 15.10.2019
(51) Int. Cl.: A61N 7/00, A61F 13/00, A61F 13/02, A61M 1/00

(54) **TISSUE TREATMENT DEVICE**
GEWEBEBEHANDLUNGSVORRICHTUNG
DISPOSITIF DE TRAITEMENT DES TISSUS

(30) Priority: 18.10.2018 GB 201816971; 08.05.2019 GB 201906451
(43) Date of publication of application: 25.08.2021
(73) Proprietor: T.J.SMITH AND NEPHEW, LIMITED, Hull HU3 2BN (GB)
(72) Inventor: DAGGER, Anthony, Hull HU3 2BN (GB); HARTWELL, Edward, Yerbury, Hull HU3 2BN (GB); RAWSON, Neill, John, Hull HU3 2BN (GB); WEBSTER, Iain, Hull HU3 2BN (GB)
(74) Representative: Appleyard Lees IP LLP
(86) International application number: PCT/EP2019/077947
(87) International publication number: WO 2020/078993

(56) References cited:
- EP-A1- 2 571 467
- EP-B1- 2 571 467
- WO-A1-99/56829
- WO-A2-2006/114638
- WO-A2-2007/030601
- WO-A2-2007/084792
- US-A1- 2014 200 487

## Description

### BACKGROUND

### Technical Field

Embodiments described herein relate to apparatuses and systems for the treatment of wounds with ultrasound.

### Description of the Related Art

Chronic wounds and/or open wounds that do not heal within a normal timeframe are a significant problem amongst certain patient populations, particularly older patients that may have a compromised vasculature. Treatment of such wounds via application of negative pressure wound therapy (NPWT) to the wound site is well known in the art and numerous therapies exist. For example, NPWT systems and dressings such as PICO, RENASYS-F, RENASYS-G, RENASYS-AB, RENASYS-F/AB are currently available from Smith & Nephew.

The use of therapeutic ultrasound for the treatment of bone is also well known in the art. For example, the EXOGEN Ultrasound Bone Healing device by Bioventus is FDA-approved for accelerating the healing of bone fractures. However, application of therapeutic ultrasound to wounded tissue outside of bone is not well-known in the art, nor is the application of therapeutic ultrasound in combination with NPWT. Application of ultrasound to wounded tissue or unwounded tissue presents a number of complications, such as the proper mode of delivery and ideal signal parameters for healing intact and/or wounded tissue. Consequently, a proper vehicle for the delivery of therapeutic ultrasound to wounds is currently unknown.

Many different types of dressings are known in the art for use in wound healing. These different types of wound dressings include many different types of materials and layers, for example, gauze, pads, foam pads or multi-layer wound dressings. However, use of such dressings in combination with therapeutic ultrasound is not well-understood in the art, particularly due to the difficulties in transmitting therapeutic ultrasound through various mediums into tissue. Additionally, the optimum signal parameters for wound healing or for treating other types of tissues is not well-known. Prior art apparatuses are known from WO 2006/114638 A2. WO 2007/084792 A2, WO 99/56829 A1, US 2014/200487 A1, WO 2007/030601 A2, and EP 2 571 467 A1.

Therefore, improved apparatuses for delivering ultrasound to wounds and other tissues are needed.

### SUMMARY

Certain disclosed embodiments relate to devices and systems for monitoring tissues. It will be understood by one of skill in the art that application of the devices and systems described herein is not limited to a particular tissue or a particular injury. The invention is defined by independent claim 1. Preferred embodiments are set out in the dependent claims.

In certain embodiments, a therapeutic ultrasound wound treatment apparatus comprises:
a wound contact layer;
a delivery layer positioned over the wound contact layer, the delivery layer comprising a porous portion and a transmission portion, the transmission portion comprising a transmission material configured to transmit vibrational energy;
an ultrasonic transducer, the ultrasonic transducer positioned over the transmission portion, the ultrasonic transducer configured to deliver vibrational energy at a therapeutic frequency;
an absorbent layer positioned over the delivery layer, the absorbent layer configured to absorb wound exudate; and
a cover layer overlying the absorbent layer; and the transmission portion extends through an entire thickness of the delivery layer.

In certain embodiments, the transmission portion may comprise silicone gel. In some embodiments, the transmission portion may extend through an entire thickness of the delivery layer. The ultrasonic transducer may comprise a piezoelectric transducer. A fluid connector may be attached to the cover layer, the fluid connector in communication with a space under the wound cover. In certain embodiments, the fluid connector may be configured to be in fluid communication with a source of negative pressure. In some embodiments of the apparatuses disclosed above, there may be a plurality of ultrasonic transducers and a plurality of transmission portions, each ultrasonic transducer positioned over a transmission portion and configured to deliver vibrational energy at a therapeutic frequency. The plurality of ultrasonic transducers may comprise five ultrasonic transducers, the five ultrasonic transducers arranged in a cross pattern.

In some embodiments, a therapeutic ultrasound wound treatment apparatus may comprise:
a wound contact layer, the wound contact layer configured to transmit vibrational energy;
an ultrasonic transducer positioned above the wound contact layer, the ultrasonic transducer configured to deliver vibrational energy at a therapeutic frequency; and
a cover layer positioned over the ultrasonic transducer.

In certain embodiments, a therapeutic ultrasound wound dressing may comprise:
a wound contact layer;
a delivery layer positioned over the wound contact layer, the foam delivery layer comprising a central through hole;
an absorbent layer positioned over the delivery layer and comprising a central through hole, the absorbent layer configured to absorb wound exudate;
a column oftransmission material extending through the foam delivery layer and the absorbent layer, the transmission material configured to transmit vibrational energy; and
a cover layer overlying the absorbent layer.

The delivery layer may comprise foam. In certain embodiments, the vibrational energy may be controlled by a controller, the controller configured to apply vibrational energy at a frequency of 1.5 MHz.

In some embodiments, a therapeutic ultrasound wound treatment apparatus may comprise a substantially flexible integrated substrate with a first wound-facing side comprising an plurality of ultrasonic transducers and a second side opposite the first side, the ultrasonic transducers configured to deliver vibrational energy at a therapeutic frequency, and a substantially flexible protective coating encapsulating the substantially flexible integrated substrate, the substantially flexible protective coating comprising a plurality of perforations configured to facilitate passage of fluid.

In certain embodiments, the plurality of ultrasonic transducers may be arranged in an array. The substantially flexible protective coating may comprise silicone. The perforations may comprise wicking cores, the wicking cores configured to wick fluid. The substantially flexible integrated substrate may further comprise a plurality of electrical connections, the electrical connections coated in a stress-resistant hard coat.

In some embodiments, a therapeutic ultrasound treatment system may comprise a therapeutic ultrasound wound treatment apparatus as described above; and an ultrasound transmission wound filler configured to be positioned beneath the wound treatment apparatus. The ultrasound transmission wound filler may comprise a plurality of perforations. In embodiments, the ultrasound transmission wound filler perforations may be configured to be aligned with the plurality of perforations in the therapeutic ultrasound wound treatment apparatus. The ultrasound transmission wound filler perforations may comprise a first size and the therapeutic ultrasound wound treatment apparatus perforations may comprise a second size. The system may further comprise a wicking layer, the wicking layer configured to be positioned beneath the ultrasound transmission wound filler. The wicking layer may be a net. The wicking layer may be configured to fold over and cover the ultrasound transmission wound filler. In some embodiments, the therapeutic ultrasound treatment system may comprise wicking fingers, the wicking fingers configured to be positioned around the perimeter of the ultrasound transmission wound filler. In some embodiments, a therapeutic ultrasound wound treatment apparatus may comprise: a cover layer overlying a therapeutic ultrasonic transducer array, the ultrasonic transducer array configured to deliver vibrational energy at a therapeutic frequency; a first wicking layer positioned beneath the transducer array; a first wound contact layer positioned beneath the first wicking layer; a transmission wound filler positioned beneath the wound contact layer, the transmission wound filler overlying a second wicking layer; and a second wound contact layer, the second wound contact layer positioned beneath the second wicking layer.

In certain embodiments, a therapeutic ultrasound wound treatment apparatus may comprise one or more features of the foregoing description.

### BRIEF DESCRIPTION OF THE DRAWINGS

FIG. 1 illustrates a reduced pressure wound therapy system according to some embodiments.
FIG. 2A illustrates an embodiment of a negative pressure wound treatment system employing a flexible fluidic connector and a wound dressing capable of absorbing and storing wound exudate.
FIG. 2B illustrates an embodiment of a negative pressure wound treatment system employing a flexible fluidic connector and a wound dressing capable of absorbing and storing wound exudate.
FIG. 3A illustrates an embodiment of a negative pressure wound treatment system employing a flexible fluidic connector and a wound dressing capable of absorbing and storing wound exudate.
FIG. 3B illustrates a cross section of an embodiment of a fluidic connector connected to a wound dressing.
FIG. 4 illustrates an embodiment of a negative pressure wound therapy system.
FIG. 5 illustrates a schematic of an embodiment of an ultrasound dependent signaling pathway.
FIG. 6 illustrates an embodiment of a therapeutic ultrasound wound treatment apparatus.
FIG. 7 is a photograph of an embodiment of a therapeutic ultrasound wound treatment apparatus.
FIG. 8A illustrates an embodiment of a therapeutic ultrasound wound treatment apparatus.
FIGS. 8B-C are photographs of therapeutic ultrasound wound treatment apparatuses.
FIG. 9 illustrates embodiments of arrangements of ultrasonic transducers.
FIGS. 10A-B are photographs of therapeutic ultrasound wound treatment apparatuses.
FIG. 11 illustrates an embodiment of a dressing that may be used with ultrasound.
FIG. 12 illustrates an embodiment of an ultrasonic foot bath.
FIGS. 13A-B illustrate embodiments of calibration curves and the accompany data sets for therapeutic ultrasound apparatuses.
FIG. 14 is a figure depicting the results of a non-limiting experiment involving therapeutic ultrasound.
FIG. 15 is a figure depicting the results of a non-limiting experiment involving therapeutic ultrasound.
FIG. 16 is a figure depicting the results of a non-limiting experiment involving therapeutic ultrasound.
FIG. 17 is a figure depicting the results of a non-limiting experiment involving inhibition of the cellular response to therapeutic ultrasound.
FIG. 18 is a figure depicting the results of a non-limiting experiment involving inhibition of the cellular response to therapeutic ultrasound.
FIG. 19 illustrates the results of a non-limiting experiment involving migration of cells in response to therapeutic ultrasound.
FIG. 20 illustrates the results of a non-limiting experiment involving migration of cells in response to therapeutic ultrasound.
FIG. 21 is a figure depicting the results of a non-limiting experiment involving inhibition of the cellular response to therapeutic ultrasound.
FIG. 22 illustrates the results of a non-limiting experiment involving the effect of duration of therapeutic ultrasound therapy on increases in temperature.
FIG. 23 illustrates a testing setup to evaluate ultrasonic transmission through a film.
FIG. 24 illustrates the results of a non-limiting experiment to assess the relative increase in ultrasound transmission through various film mediums with increased drive voltage.
FIG. 25 illustrates the results of a non-limiting experiment to assess the relative increase in ultrasound transmission through various film mediums with increased drive voltage.
FIGS. 26A and 26B illustrate cross sections of embodiments of therapeutic ultrasound wound treatment apparatuses.
FIGS. 27A-27C show embodiments of therapeutic ultrasound wound treatment apparatuses. FIG. 27C is a photograph of a therapeutic ultrasound wound treatment apparatus placed on a foot.
FIGS. 28A-28B show embodiments of therapeutic ultrasound wound treatment systems including therapeutic ultrasound wound treatment apparatuses.
FIGS. 29A-29C show embodiments of a therapeutic ultrasound wound treatment apparatus. FIGS. 29B-29C are photographs of transducer arrays that may be part of said therapeutic ultrasound wound treatment apparatus embodiments.
FIG. 30 illustrates an embodiment of a wound interface for a therapeutic wound treatment apparatus.

### DETAILED DESCRIPTION

Embodiments disclosed herein relate to apparatuses and methods for treating wounds and other tissues with therapeutic ultrasound, either with or without NPWT. The embodiments disclosed herein are not limited to treatment of a particular type of tissue or injury, instead the therapeutic ultrasound technologies disclosed herein are broadly applicable to any type of wounded or intact tissue that may benefit from therapeutic ultrasound. For example, the therapeutic ultrasound embodiments disclosed herein may be used to treat both internal and external wounds. Some embodiments disclosed herein relate to the use of therapeutic ultrasound delivered alone or in combination with a material layer (such as a dressing) configured to be used in the treatment of both intact and damaged human or animal tissue. The therapeutic ultrasound embodiments disclosed herein may be used with any of the signal parameters, such as intensity, timing, and frequency disclosed herein this section or elsewhere in the specification. Further details regarding the arrangement of a therapeutic ultrasound system within a dressing and optimization of the therapeutic ultrasound signal will be described in much greater detail later in the specification. Further details regarding the impact of treatment with therapeutic ultrasound may be found in "Ultrasonic Stimulation of Mouse Skin Reverses the Healing Delays in Diabetes and Aging by Activation of Rac1," by Roper et al., "Therapeutic Ultrasound Bypasses Canonical Syndecan-4 Signaling to Activate Rac1" by Mahoney et al., and "Cytoplasmic interactions of syndecan-4 orchestrate adhesion receptor and growth factor receptor signaling" by Bass and Humphries. Further details regarding the cellular impact of therapeutic ultrasound may be found in "Induction of Adhesion-dependent Signals Using Low-Intensity Ultrasound" by Roper et al.

The therapeutic ultrasound embodiments disclosed herein may be used in combination with clothing; for example, by incorporating an ultrasound transducer within layers of clothing. Non-limiting examples of clothing for use with therapeutic ultrasound disclosed herein include shirts, pants, trousers, dresses, undergarments, outer-garments, gloves, shoes, hats, and other suitable garments. The therapeutic ultrasound embodiments disclosed herein may be incorporated into cushioning or bed padding, such as within a hospital bed, to monitor patient characteristics, such as any characteristic disclosed herein. In certain embodiments, a disposable film containing such sensors could be placed over the hospital bedding and removed/replaced as needed. The therapeutic ultrasound embodiments disclosed herein may be utilized in rehabilitation devices and treatments, including sports medicine. For example, the therapeutic ultrasound embodiments disclosed herein may be used in braces, sleeves, wraps, supports, and other suitable items.

The therapeutic ultrasound embodiments disclosed herein may be incorporated into implantable devices, such as implantable orthopedic implants, including flexible implants. Such embodiments may be configured to treat tissue surrounding the implant site. In some embodiments, an internal source may also provide power for such an implant.

Therapeutic ultrasound embodiments as disclosed herein may be incorporated into Ear, Nose, and Throat (ENT) applications. For example, such Therapeutic ultrasound embodiments may be utilized to treat the tissues of the passages of the ear, nose and throat.

In certain embodiments, the therapeutic ultrasound embodiments disclosed herein may be incorporated into an organ protection layer such as disclosed below. Such a therapeutic ultrasound incorporated organ protection layer may both protect the organ of interest and treat the underlying tissues and organs. As discussed above, the therapeutic ultrasound embodiments disclosed herein may be incorporated into treatments for wounds (disclosed in greater detail below) or in a variety of other applications. Non-limiting examples of additional applications for the therapeutic ultrasound embodiments disclosed herein include: treatment of intact skin, cardiovascular applications such as delivering therapeutic ultrasound to blood vessels, orthopedic applications such as delivering therapeutic ultrasound to intact and fractures bone and other skeletal tissues, neurophysiological applications such as delivering therapeutic ultrasound to the central and peripheral nervous system, and any other tissue, organ, system, or condition that may benefit from therapeutic ultrasound.

### Wound Therapy

Some embodiments disclosed herein relate to wound therapy for a human or animal body. Therefore, any reference to a wound herein can refer to a wound on a human or animal body, and any reference to a body herein can refer to a human or animal body. The disclosed technology embodiments may relate to preventing or minimizing damage to physiological tissue or living tissue, or to the treatment of damaged tissue (for example, a wound as described herein) wound with or without reduced pressure, including for example a source of negative pressure and wound dressing components and apparatuses.

The apparatuses and components comprising the wound overlay and packing materials or internal layers, if any, are sometimes collectively referred to herein as dressings. In some embodiments, the wound dressing can be provided to be utilized without reduced pressure.

Some embodiments disclosed herein relate to wound therapy for a human or animal body. Therefore, any reference to a wound herein can refer to a wound on a human or animal body, and any reference to a body herein can refer to a human or animal body. The disclosed technology embodiments may relate to preventing or minimizing damage to physiological tissue or living tissue, or to the treatment of damaged tissue (for example, a wound as described herein). Methods of treatment by therapy or surgery disclosed herein are not part of the claimed invention.

As used herein the expression "wound" may include an injury to living tissue may be caused by a cut, blow, or other impact, typically one in which the skin is cut or broken. A wound may be a chronic or acute injury. Acute wounds occur as a result of surgery or trauma. They move through the stages of healing within a predicted timeframe. Chronic wounds typically begin as acute wounds. The acute wound can become a chronic wound when it does not follow the healing stages resulting in a lengthened recovery. It is believed that the transition from acute to chronic wound can be due to a patient being immuno-compromised.

Chronic wounds may include for example: venous ulcers (such as those that occur in the legs), which account for the majority of chronic wounds and mostly affect the elderly, diabetic ulcers (for example, foot or ankle ulcers), peripheral arterial disease, pressure ulcers, or epidermolysis bullosa (EB).

Examples of other wounds include, but are not limited to, abdominal wounds or other large or incisional wounds, either as a result of surgery, trauma, sterniotomies, fasciotomies, or other conditions, dehisced wounds, acute wounds, chronic wounds, subacute and dehisced wounds, traumatic wounds, flaps and skin grafts, lacerations, abrasions, contusions, bums, diabetic ulcers, pressure ulcers, stoma, surgical wounds, trauma and venous ulcers or the like.

Wounds may also include a deep tissue injury. Deep tissue injury is a term proposed by the National Pressure Ulcer Advisory Panel (NPUAP) to describe a unique form of pressure ulcers. These ulcers have been described by clinicians for many years with terms such as purple pressure ulcers, ulcers that are likely to deteriorate and bruises on bony prominences.

Wound may also include tissue at risk of becoming a wound as discussed herein. For example, tissue at risk may include tissue over a bony protuberance (at risk of deep tissue injury/insult) or pre-surgical tissue (for example, knee tissue) that may has the potential to be cut (for example, for joint replacement/surgical alteration/reconstruction).

Some examples relate to methods, not forming part of the claimed invention, of treating a wound with the technology disclosed herein in conjunction with one or more of the following: advanced footwear, turning a patient, offloading (such as, offloading diabetic foot ulcers), treatment of infection, systemix, antimicrobial, antibiotics, surgery, removal of tissue, affecting blood flow, physiotherapy, exercise, bathing, nutrition, hydration, nerve stimulation, ultrasound, electrostimulation, oxygen therapy, microwave therapy, active agents ozone, antibiotics, antimicrobials, or the like.

Alternatively or additionally, a wound may be treated using topical negative pressure and/or traditional advanced wound care, which is not aided by the using of applied negative pressure (may also be referred to as non-negative pressure therapy).

Advanced wound care may include use of an absorbent dressing, an occlusive dressing, use of an antimicrobial and/or debriding agents in a wound dressing or adjunct, a pad (for example, a cushioning or compressive therapy, such as stockings or bandages), or the like.

In some embodiments, treatment of such wounds can be performed using traditional wound care, wherein a dressing can be applied to the wound to facilitate and promote healing of the wound.

Some embodiments relate to methods of manufacturing a wound dressing comprising providing a wound dressing as disclosed herein.

The wound dressings that may be utilized in conjunction with the disclosed technology include any known dressing in the art. The technology is applicable to negative pressure therapy treatment as well as non-negative pressure therapy treatment.

In some embodiments, a wound dressing comprises one or more absorbent layer(s). The absorbent layer may be a foam or a superabsorbent.

In some embodiments, wound dressings may comprise a dressing layer including a polysaccharide or modified polysaccharide, a polyvinylpyrrolidone, a polyvinyl alcohol, a polyvinyl ether, a polyurethane, a polyacrylate, a polyacrylamide, collagen, or gelatin or mixtures thereof. Dressing layers comprising the polymers listed are known in the art as being useful for forming a wound dressing layer for either negative pressure therapy or non-negative pressure therapy.

In some embodiments, the polymer matrix may be a polysaccharide or modified polysaccharide.

In some embodiments, the polymer matrix may be a cellulose. Cellulose material may include hydrophilically modified cellulose such as methyl cellulose, carboxymethyl cellulose (CMC), carboxymethyl cellulose (CEC), ethyl cellulose, propyl cellulose, hydroxyethyl cellulose, hydroxypropyl cellulose, hydroxypropylmethyl cellulose, carboxyethyl sulphonate cellulose, cellulose alkyl sulphonate, or mixtures thereof.

In certain embodiments, cellulose material may be cellulose alkyl sulphonate. The alkyl moiety of the alkyl sulphonate substituent group may have an alkyl group having 1 to 6 carbon atoms, such as methyl, ethyl, propyl, or butyl. The alkyl moiety may be branched or unbranched, and hence suitable propyl sulphonate substituents may be 1- or 2-methyl-ethylsulphonate. Butyl sulphonate substituents may be 2-ethyl-ethylsulphonate, 2,2-dimethyl-ethylsulphonate, or 1,2-dimethyl-ethylsulphonate. The alkyl sulphonate substituent group may be ethyl sulphonate. The cellulose alkyl sulphonate is described in WO10061225, US2016/114074, US2006/0142560, or US 5,703,225.

Cellulose alkyl sulfonates may have varying degrees of substitution, the chain length of the cellulose backbone structure, and the structure of the alkyl sulfonate substituent. Solubility and absorbency are largely dependent on the degree of substitution: as the degree of substitution is increased, the cellulose alkyl sulfonate becomes increasingly soluble. It follows that, as solubility increases, absorbency increases.

In some embodiments, a wound dressing also comprises a top or cover layer. The thickness of the wound dressing disclosed herein may be between 1 to 20, or 2 to 10, or 3 to 7 mm.

In some embodiments, the disclosed technology may be used in conjunction with a non-negative pressure dressing. A non-negative pressure wound dressing suitable for providing protection at a wound site may comprise:
an absorbent layer for absorbing wound exudate and
an obscuring element for at least partially obscuring a view of wound exudate absorbed by the absorbent layer in use.

The obscuring element may be partially translucent, and in certain embodiments the obscuring element may be a masking layer.

The non-negative pressure wound dressing may further comprise a region in or adjacent the obscuring element for allowing viewing of the absorbent layer. For example, the obscuring element layer may be provided over a central region of the absorbent layer and not over a border region of the absorbent layer. In some embodiments, the obscuring element is of hydrophilic material or is coated with a hydrophilic material.

The obscuring element may comprise a three-dimensional knitted spacer fabric. The spacer fabric is known in the art and may include a knitted spacer fabric layer.

The obscuring element may further comprise an indicator for indicating the need to change the dressing.

In some embodiments, the obscuring element is provided as a layer at least partially over the absorbent layer, further from a wound site than the absorbent layer in use.

The non-negative pressure wound dressing may further comprise a plurality of openings in the obscuring element for allowing fluid to move therethrough. The obscuring element may comprise, or may be coated with, a material having size-exclusion properties for selectively permitting or preventing passage of molecules of a predetermined size or weight.

The obscuring element may be configured to at least partially mask light radiation having wavelength of 600 nm and less.

The obscuring element may be configured to reduce light absorption by 50% or more.

The obscuring element may be configured to yield a CIE L* value of 50 or more, and optionally 70 or more. In some embodiments, the obscuring element may be configured to yield a CIE L* value of 70 or more.

In some embodiments, the non-negative pressure wound dressing may further comprise at least one of a wound contact layer, a foam layer, an odor control element, a pressure-resistant layer and a cover layer.

In some embodiments, the cover layer is present, and the cover layer is a translucent film. Typically, the translucent film has a moisture vapour permeability of 500g/m2/24hours or more.

The translucent film may be a bacterial barrier.

In some embodiments, the non-negative pressure wound dressing as disclosed herein comprises the wound contact layer and the absorbent layer overlies the wound contact layer. The wound contact layer carries an adhesive portion for forming a substantially fluid tight seal over the wound site.

The non-negative pressure wound dressing as disclosed herein may comprise the obscuring element and the absorbent layer being provided as a single layer.

In some embodiments, the non-negative pressure wound dressing disclosed herein comprises the foam layer, and the obscuring element is of a material comprising components that may be displaced or broken by movement of the obscuring element.

In some embodiments, the non-negative pressure wound dressing comprises an odor control element, and in another embodiment the dressing does not include an odor control element. When present, the odor control element may be dispersed within or adjacent the absorbent layer or the obscuring element. Alternatively, when present the odor control element may be provided as a layer sandwiched between the foam layer and the absorbent layer.

In some embodiments, the disclosed technology for a non-negative pressure wound dressing comprises a method of manufacturing a wound dressing, comprising: providing an absorbent layer for absorbing wound exudate; and providing an obscuring element for at least partially obscuring a view of wound exudate absorbed by the absorbent layer in use.

In some embodiments, the non-negative pressure wound dressing is may be suitable for providing protection at a wound site, comprising: an absorbent layer for absorbing wound exudate; and a shielding layer provided over the absorbent layer, and further from a wound-facing side of the wound dressing than the absorbent layer. The shielding layer may be provided directly over the absorbent layer. In some embodiments, the shielding layer comprises a three-dimensional spacer fabric layer.

The shielding layer increases the area over which a pressure applied to the dressing is transferred by 25% or more or the initial area of application. For example the shielding layer increases the area over which a pressure applied to the dressing is transferred by 50% or more, and optionally by 100% or more, and optionally by 200% or more.

The shielding layer may comprise 2 or more sub-layers, wherein a first sub-layer comprises through holes and a further sub-layer comprises through holes and the through holes of the first sub-layer are offset from the through holes of the further sub-layer.

The non-negative pressure wound dressing as disclosed herein may further comprise a permeable cover layer for allowing the transmission of gas and vapour therethrough, the cover layer provided over the shielding layer, wherein through holes of the cover layer are offset from through holes of the shielding layer.

The non-negative pressure wound dressing may be suitable for treatment of pressure ulcers.

A more detailed description of the non-negative pressure dressing disclosed hereinabove is provided in WO2013/007973.

In some embodiments, the non-negative pressure wound dressing may be a multi-layered wound dressing comprising: a fibrous absorbent layer for absorbing exudate from a wound site; and a support layer configured to reduce shrinkage of at least a portion of the wound dressing.

In some embodiments, the multi-layered wound dressing disclosed herein, further comprises a liquid impermeable film layer, wherein the support layer is located between the absorbent layer and the film layer.

The support layer disclosed herein may comprise a net. The net may comprise a geometric structure having a plurality of substantially geometric apertures extending therethrough. The geometric structure may for example comprise a plurality of bosses substantially evenly spaced and joined by polymer strands to form the substantially geometric apertures between the polymer strands.

The net may be formed from high density polyethylene. The apertures may have an area from 0.005 to 0.32 mm2. The support layer may have a tensile strength from 0.05 to 0.06 Nm. The support layer may have a thickness of from 50 to 150 µm.

In some embodiments, the support layer is located directly adjacent the absorbent layer. Typically, the support layer is bonded to fibers in a top surface of the absorbent layer. The support layer may further comprise a bonding layer, wherein the support layer is heat laminated to the fibers in the absorbent layer via the bonding layer. The bonding layer may comprise a low melting point adhesive such as ethylene-vinyl acetate adhesive.

In some embodiments, the multi-layered wound dressing disclosed herein further comprises an adhesive layer attaching the film layer to the support layer.

In some embodiments, the multi-layered wound dressing disclosed herein further comprises a wound contact layer located adjacent the absorbent layer for positioning adjacent a wound. The multi-layered wound dressing may further comprise a fluid transport layer between the wound contact layer and the absorbent layer for transporting exudate away from a wound into the absorbent layer.

A more detailed description of the multi-layered wound dressing disclosed hereinabove is provided in PCT patent publication WO2018078 72.

In some embodiments, the disclosed technology may be incorporated in a wound dressing comprising a vertically lapped material comprising: a first layer of an absorbing layer of material, and a second layer of material, wherein the first layer being constructed from at least one layer of non-woven textile fibers, the non-woven textile fibers being folded into a plurality of folds to form a pleated structure. In some embodiments, the wound dressing further comprises a second layer of material that is temporarily or permanently connected to the first layer of material.

Typically the vertically lapped material has been slitted.

In some embodiments, the first layer has a pleated structure having a depth determined by the depth of pleats or by the slitting width. The first layer of material may be a moldable, lightweight, fiber-based material, blend of material or composition layer.

The first layer of material may comprise one or more of manufactured fibers from synthetic, natural or inorganic polymers, natural fibers of a cellulosic, proteinaceous or mineral source.

The wound dressing may comprise two or more layers of the absorbing layer of material vertically lapped material stacked one on top of the other, wherein the two or more layers have the same or different densities or composition.

The wound dressing may in some embodiments comprise only one layer of the absorbing layer of material vertically lapped material.

The absorbing layer of material is a blend of natural or synthetic, organic or inorganic fibers, and binder fibers, or bicomponent fibers typically PET with a low melt temperature PET coating to soften at specified temperatures and to act as a bonding agent in the overall blend.

In some embodiments, the absorbing layer of material may be a blend of 5 to 95 % thermoplastic polymer, and 5 to 95 wt % of a cellulose or derivative thereof.

In some embodiments, the wound dressing disclosed herein has a second layer comprises a foam or a dressing fixative.

The foam may be a polyurethane foam. The polyurethane foam may have an open or closed pore structure.

The dressing fixative may include bandages, tape, gauze, or backing layer.

In some embodiments, the wound dressing as disclosed herein comprises the absorbing layer of material connected directly to a second layer by lamination or by an adhesive, and the second layer is connected to a dressing fixative layer. The adhesive may be an acrylic adhesive, or a silicone adhesive.

In some embodiments, the wound dressing as disclosed herein further comprises layer of a superabsorbent fiber, or a viscose fiber or a polyester fiber.

In some embodiments, the wound dressing as disclosed herein further comprises a backing layer. The backing layer may be a transparent or opaque film. Typically the backing layer comprises a polyurethane film (typically a transparent polyurethane film).

A more detailed description of the multi-layered wound dressing disclosed hereinabove is provided in GB patent applications filed on 12 December 2016 with application number GB1621057.7; and 22 June 2017 with application number GB1709987.0.

In some embodiments, the non-negative pressure wound dressing may comprise an absorbent component for a wound dressing, the component comprising a wound contacting layer comprising gel forming fibers bound to a foam layer, wherein the foam layer is bound directly to the wound contact layer by an adhesive, polymer based melt layer, by flame lamination or by ultrasound.

The absorbent component may be in a sheet form.

The wound contacting layer may comprise a layer of woven or non-woven or knitted gel forming fibers.

The foam layer may be an open cell foam, or closed cell foam, typically an open cell foam. The foam layer is a hydrophilic foam.

The wound dressing may comprise the component that forms an island in direct contact with the wound surrounded by periphery of adhesive that adheres the dressing to the wound. The adhesive may be a silicone or acrylic adhesive, typically a silicone adhesive.

The wound dressing may be covered by a film layer on the surface of the dressing furthest from the wound.

A more detailed description of the wound dressing of this type hereinabove is provided in PCT publication WO2011058311A1.

In some embodiments, the non-negative pressure wound dressing may comprise a multi layered wound dressing for use on wounds producing high levels of exudate, characterized in that the dressing comprising: a transmission layer having an MVTR of at least 300 gm2/24 hours, an absorbent core comprising gel forming fibers capable of absorbing and retaining exudate, a wound contacting layer comprising gel forming fibers which transmits exudate to the absorbent core and a keying layer positioned on the absorbent core, the absorbent core and wound contacting layer limiting the lateral spread of exudate in the dressing to the region of the wound.

The wound dressing may be capable of handling at least 6g (or 8g and 15g) of fluid per 10cm2 of dressing in 24 hours.

The wound dressing may comprise gel forming fibers that are chemically modified cellulosic fibers in the form of a fabric. The fibers may include carboxymethylated cellulose fibers, typically sodium carboxymethylcellulose fiber.

The wound dressing may comprise a wound contact layer with a lateral wicking rate from 5mm per minute to 40mm per minute. The wound contact layer may have a fiber density between 25gm2 and 55gm2, such as 35gm2.

The absorbent core may have an absorbency of exudate of at least 10g/g, and typically a rate of lateral wicking of less the 20mm per minute.

The absorbent core may have a blend in the range of up to 25% cellulosic fibers by weight and 75% to 100% gel forming fibers by weight.

Alternatively, the absorbent core may have a blend in the range of up to 50% cellulosic fibers by weight and 50% to 100% gel forming fibers by weight. For example the blend is in the range of 50% cellulosic fibers by weight and 50% gel forming fibers by weight.

The fiber density in the absorbent core may be between 150gm2 and 250gm2, or about 200 gm2.

The wound dressing when wet may have shrinkage that is less than 25 % or less than 15 % of its original size/dimension.

The wound dressing may comprise a transmission layer and the layer is a foam. The transmission layer may be a polyurethane foam laminated to a polyurethane film.

The wound dressing may comprise one or more layers selected from the group comprising a soluble medicated film layer; an odor-absorbing layer; a spreading layer and an additional adhesive layer.

The wound dressing may be 2mm and 4mm thick.

The wound dressing may be characterized in that the keying layer bonds the absorbent core to a neighboring layer. In some embodiments, the keying layer may be positioned on either the wound facing side of the absorbent core or the non-wound facing side of the absorbent core. In some embodiments, the keying layer is positioned between the absorbent core and the wound contact layer. The keying layer is a polyamide web.

A more detailed description of the wound dressing of this type hereinabove is provided in PCT Publication WO2005079718A1.

In some embodiments, the non-negative pressure wound dressing may be a compression bandage. Compression bandages are known for use in the treatment of oedema and other venous and lymphatic disorders, e.g., of the lower limbs.

A compression bandage systems typically employ multiple layers including a padding layer between the skin and the compression layer or layers. The compression bandage may be useful for wounds such as handling venous leg ulcers.

The compression bandage in some embodiments may comprise a bandage system comprising an inner skin facing layer and an elastic outer layer, the inner layer comprising a first ply of foam and a second ply of an absorbent nonwoven web, the inner layer and outer layer being sufficiently elongated so as to be capable of being wound about a patient's limb. A compression bandage of this type is disclosed in WO99/58090.

In some embodiments, the compression bandage system comprises: a) an inner skin facing, elongated, elastic bandage comprising: (i) an elongated, elastic substrate, and (ii) an elongated layer of foam, said foam layer being affixed to a face of said substrate and extending 33% or more across said face of substrate in transverse direction and 67% or more across said face of substrate in longitudinal direction; and b) an outer, elongated, self- adhering elastic bandage; said bandage having a compressive force when extended; wherein, in use, said foam layer of the inner bandage faces the skin and the outer bandage overlies the inner bandage. A compression bandage of this type is disclosed in WO2006/110527.

In some embodiments other compression bandage systems such as those disclosed in US 6,759,566 and US 2002/0099318.

### Negative Pressure Wound Therapy Dressings

In some embodiments, treatment of wounds can be performed using negative pressure wound therapy, wherein a reduced or negative pressure can be applied to the wound to facilitate and promote healing of the wound. It will also be appreciated that the wound dressing and methods as disclosed herein may be applied to other parts of the body, and are not necessarily limited to treatment of wounds. One of skill in the art will understand that the NPWT embodiments disclosed herein may be combined with any of the therapeutic ultrasound embodiments described herein to provide simultaneous or alternating therapeutic ultrasound and negative pressure.

It will be understood that embodiments of the present disclosure are generally applicable to use in topical negative pressure ("TNP") or negative pressure wound therapy (NPWT) systems. Briefly, negative pressure wound therapy assists in the closure and healing of many forms of "hard to heal" wounds by reducing tissue oedema; encouraging blood flow and granular tissue formation; removing excess exudate and may reduce bacterial load (and thus infection risk). In addition, the therapy allows for less disturbance of a wound leading to more rapid healing.

TNP therapy systems may also assist on the healing of surgically closed wounds by removing fluid and by helping to stabilize the tissue in the apposed position of closure. A further beneficial use of TNP therapy can be found in grafts and flaps where removal of excess fluid is important and close proximity of the graft to tissue is required in order to ensure tissue viability.

Negative pressure therapy can be used for the treatment of open or chronic wounds that are too large to spontaneously close or otherwise fail to heal by means of applying negative pressure to the site of the wound. Topical negative pressure (TNP) therapy or negative pressure wound therapy (NPWT) involves placing a cover that is impermeable or semi-permeable to fluids over the wound, using various means to seal the cover to the tissue of the patient surrounding the wound, and connecting a source of negative pressure (such as a vacuum pump) to the cover in a manner so that negative pressure is created and maintained under the cover. It is believed that such negative pressures promote wound healing by facilitating the formation of granulation tissue at the wound site and assisting the body's normal inflammatory process while simultaneously removing excess fluid, which may contain adverse cytokines or bacteria.

Some of the dressings used in NPWT can include many different types of materials and layers, for example, gauze, pads, foam pads or multi-layer wound dressings. One example of a multi-layer wound dressing is the PICO dressing, available from Smith & Nephew, includes a wound contact layer and a superabsorbent layer beneath a backing layer to provide a canister-less system for treating a wound with NPWT. The wound dressing may be sealed to a suction port providing connection to a length of tubing, which may be used to pump fluid out of the dressing or to transmit negative pressure from a pump to the wound dressing. Additionally, RENASYS-F, RENASYS-G, RENASYS-AB, and RENASYS-F/AB, available from Smith & Nephew, are additional examples of NPWT wound dressings and systems. Another example of a multi-layer wound dressing is the ALLEVYN Life dressing, available from Smith & Nephew, which includes a moist wound environment dressing that is used to treat the wound without the use of negative pressure.

As is used herein, reduced or negative pressure levels, such as -X mmHg, represent pressure levels relative to normal ambient atmospheric pressure, which can correspond to 760 mmHg (or 1 atm, 29.93 inHg, 101.325 kPa, 14.696 psi, etc.). Accordingly, a negative pressure value of -X mmHg reflects absolute pressure that is X mmHg below 760 mmHg or, in other words, an absolute pressure of (760-X) mmHg. In addition, negative pressure that is "less" or "smaller" than X mmHg corresponds to pressure that is closer to atmospheric pressure (such as,-40 mmHg is less than -60 mmHg). Negative pressure that is "more" or "greater" than -X mmHg corresponds to pressure that is further from atmospheric pressure (such as, -80 mmHg is more than -60 mmHg). In some embodiments, local ambient atmospheric pressure is used as a reference point, and such local atmospheric pressure may not necessarily be, for example, 760 mmHg.

The negative pressure range for some embodiments of the present disclosure can be approximately -80 mmHg, or between about -20 mmHg and -200 mmHg. Note that these pressures are relative to normal ambient atmospheric pressure, which can be 760 mmHg. Thus, -200 mmHg would be about 560 mmHg in practical terms. In some embodiments, the pressure range can be between about -40 mmHg and -150 mmHg. Alternatively a pressure range of up to -75 mmHg, up to -80 mmHg or over -80 mmHg can be used. Also in other embodiments a pressure range of below -75 mmHg can be used. Alternatively, a pressure range of over approximately -100 mmHg, or even -150 mmHg, can be supplied by the negative pressure apparatus.

In some embodiments of wound closure devices described herein, increased wound contraction can lead to increased tissue expansion in the surrounding wound tissue. This effect may be increased by varying the force applied to the tissue, for example by varying the negative pressure applied to the wound over time, possibly in conjunction with increased tensile forces applied to the wound via embodiments of the wound closure devices. In some embodiments, negative pressure may be varied over time for example using a sinusoidal wave, square wave, or in synchronization with one or more patient physiological indices (such as, heartbeat). Examples of such applications where additional disclosure relating to the preceding may be found include U.S. Patent No. 8,235,955, titled "Wound treatment apparatus and method," issued on August 7, 2012; and U.S. Patent No. 7,753,894, titled "Wound cleansing apparatus with stress," issued July 13, 2010.

Embodiments ofthe wound dressings, wound dressing components, wound treatment apparatuses and methods described herein may also be used in combination or in addition to those described in International Application No. PCT/IB2013/001469, filed May 22, 2013, published as WO 2013/175306 A2 on November 28, 2013, titled "APPARATUSES AND METHODS FOR NEGATIVE PRESSURE WOUND THERAPY," U.S. Patent Application No. 14/418,908, filed January 30, 2015, published as US 2015/0190286 A1 on July 9, 2015, titled "WOUND DRESSING AND METHOD OF TREATMENT". Embodiments of the wound dressings, wound dressing components, wound treatment apparatuses and methods described herein may also be used in combination or in addition to those described in U.S. Patent Application No. 13/092,042, filed April 21, 2011, published as US2011/0282309, titled "WOUND DRESSING AND METHOD OF USE," and U.S. Patent Application No. 14/715,527, filed May 18, 2015, published as US2016/0339158 A1 on November 24, 2016, titled "FLUIDIC CONNECTOR FOR NEGATIVE PRESSURE WOUND THERAPY," including further details relating to embodiments of wound dressings, the wound dressing components and principles, and the materials used for the wound dressings.

Additionally, some embodiments related to TNP wound treatment comprising a wound dressing in combination with a pump or associated electronics described herein may also be used in combination or in addition to those described in International Application PCT/EP2016/059329 filed April 26, 2016, published as WO 2016/174048 on November 3, 2016, entitled "REDUCED PRESSURE APPARATUS AND METHODS".

In some embodiments of wound closure devices described herein, increased wound contraction can lead to increased tissue expansion in the surrounding wound tissue. This effect may be increased by varying the force applied to the tissue, for example by varying the negative pressure applied to the wound over time, possibly in conjunction with increased tensile forces applied to the wound via embodiments of the wound closure devices. Further, there may be additional effects on tissues in close proximity to the filler, for example, the tissue is under compression due to the reactive force of the elastic filler pressing on the tissue. Such compression may result in in local hypoxia due to occlusion of the blood vessels. In the wider peripheral tissue, this expansion may lead to blood vessel expansion. Further details are provided in "NPWT settings and dressing choices made easy" by Malmsjo and Borgquist, published in Wounds International in May 2010. For example, in a wound that is not at risk for ischemia, the increased and decreased blood flow caused by pressure from the wound dressing is likely advantageous for wound healing. The increase in blood flow may improve oxygen and nutrient supply to the tissue, and improve penetration of antibiotics and the removal of waste. Additionally, the reduction in blood flow may stimulate angiogenesis, thereby promoting granulation tissue formation.

### Wound Healing

One of skill in the art will understand that the therapeutic ultrasound embodiments described herein are not merely applicable to situations involving wounds. Rather, such embodiments may be broadly applicable to situations that do not necessarily involve wounded tissues, such as treating intact tissues. Additionally, one of skill in the art will understand that the therapeutic ultrasound embodiments described herein may be used with any dressing embodiment described herein this section or elsewhere in the specification.

Wounds may be generally categorized as open or closed, often depending upon how the wound is caused. As described above, the techniques may be applied to both open and to closed wounds, depending on the particulars of the embodiment. Open wounds may be caused by a variety of events, including: incisions, lacerations, abrasions, punctures, pentetration, amputation, and other means. Closed wounds may be caused by damage to a blood vessel resulting in formation of a hematoma, and/or by internal injuries caused by crushing. Further, wounds may involve various layers of tissue, for example, shallower wounds may only involve the topmost layers of the skin, while deeper wounds may involve underlying subcutaneous tissue layers such as the hypodermis, including underlying connective tissues and fatty layers. In certain embodiments, wounds may even encompass underlying internal organs, deep beneath the skin. Certain wounds, such as those caused by pressure injuries, may start to occur within the deeper tissue layers without become evident on the surface of the skin until much later.

In addition to NPWT treatments described above, wounds may be treated by a wide variety of techniques and materials. For example, wounds may be treated by debridement to remove dead and/or necrotic tissue. Wounds may be treated with a with various type of dressings, including dry and wet dressings, chemically-impregnated dressings, foam dressing, hydrogel dressings, hydrocolloid dressings, film dressings, and other suitable dressings. Wounds may further be treated with bioactive molecules such as antimicrobials, growth factors, anti-inflammatories, analgesics and other suitable treatments. Such treatments may be incorporated into the aforementioned dressings.

Further details regarding wounds and wound treatment, in particular wounds caused by pressure injuries may be found in the article "Pressure Injuries (Pressure Ulcers) and Wound Care" by Kirman et al, published in Medscape March 2017.For example, the most common candidates for pressure ulcers include: elderly persons, persons who are chronically ill (such as those with cancer, stroke, or diabetes), persons who are immobile (e.g, as a consequence of fracture, arthritis, or pain), persons who are weak or debilitated, patients with altered mental status (e.g., from the effects of narcotics, anesthesia, or coma), and/or persons with decreased sensation or paralysis. Potential secondary factors include: illness or debilitation that increases pressure ulcer formation, fever (increases metabolic demands), predisposing ischemia, diaphoresis which promotes skin maceration, incontinence which causes skin irritation and contamination, edema, jaundice, pruritus, and xerosis (dry skin). Additionally, prevention of pressure ulcer injuries may include: scheduled body turning, appropriate bed positioning, protection of bony prominences, skin care, control of spascity and prevention of contractures, use of support surfaces/specialty beds, nutritional support, and maintenance of current levels of activity, mobility and range of motion.

### Negative Pressure Wound Therapy Systems

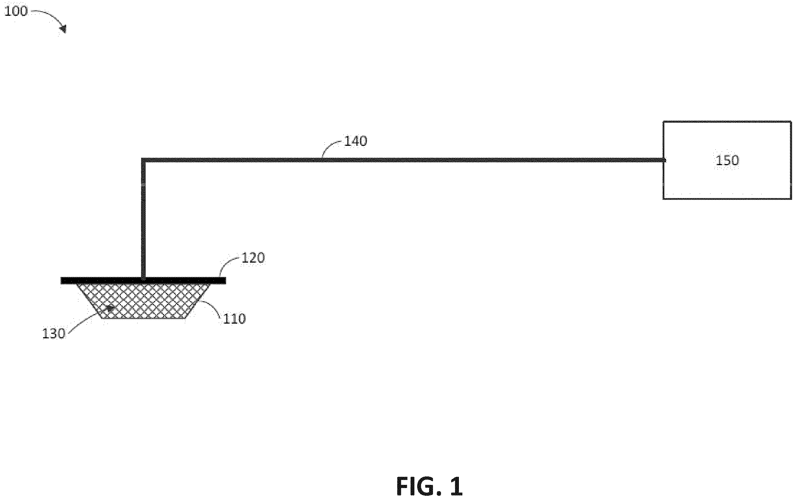

Figure 1 illustrates an embodiment of a negative or reduced pressure wound treatment (or TNP) system 100 comprising a wound filler 130 placed inside a wound cavity 110, the wound cavity sealed by a wound cover 120. The wound filler 130 in combination with the wound cover 120 can be referred to as wound dressing. A single or multi lumen tube or conduit 140 is connected the wound cover 120 with a pump assembly 150 configured to supply reduced pressure. The wound cover 120 can be in fluidic communication with the wound cavity 110. In any of the system embodiments disclosed herein, as in the embodiment illustrated in Figure 1, the pump assembly can be a canisterless pump assembly (meaning that exudate is collected in the wound dressing or is transferred via tube 140 for collection to another location). However, any of the pump assembly embodiments disclosed herein can be configured to include or support a canister. Additionally, in any of the system embodiments disclosed herein, any of the pump assembly embodiments can be mounted to or supported by the dressing, or adjacent to the dressing.

The wound filler 130 can be any suitable type, such as hydrophilic or hydrophobic foam, gauze, inflatable bag, and so on. The wound filler 130 can be conformable to the wound cavity 110 such that it substantially fills the cavity. The wound cover 120 can provide a substantially fluid impermeable seal over the wound cavity 110. The wound cover 120 can have a top side and a bottom side, and the bottom side adhesively (or in any other suitable manner) seals with wound cavity 110. The conduit 140 or lumen or any other conduit or lumen disclosed herein can be formed from polyurethane, PVC, nylon, polyethylene, silicone, or any other suitable material.

Some embodiments of the wound cover 120 can have a port (not shown) configured to receive an end of the conduit 140. For example, the port can be Renays Soft Port available from Smith & Nephew. In other embodiments, the conduit 140 can otherwise pass through or under the wound cover 120 to supply reduced pressure to the wound cavity 110 so as to maintain a target or desired level of reduced pressure in the wound cavity. The conduit 140 can be any suitable article configured to provide at least a substantially sealed fluid flow pathway between the pump assembly 150 and the wound cover 120, so as to supply the reduced pressure provided by the pump assembly 150 to wound cavity 110.

The wound cover 120 and the wound filler 130 can be provided as a single article or an integrated single unit. In some embodiments, no wound filler is provided and the wound cover by itself may be considered the wound dressing. The wound dressing may then be connected, via the conduit 140, to a source of negative pressure, such as the pump assembly 150. The pump assembly 150 can be miniaturized and portable, although larger conventional pumps such can also be used.

The wound cover 120 can be located over a wound site to be treated. The wound cover 120 can form a substantially sealed cavity or enclosure over the wound site. In some embodiments, the wound cover 120 can be configured to have a film having a high water vapor permeability to enable the evaporation of surplus fluid, and can have a superabsorbing material contained therein to safely absorb wound exudate. It will be appreciated that throughout this specification reference is made to a wound. In this sense it is to be understood that the term wound is to be broadly construed and encompasses open and closed wounds in which skin is torn, cut or punctured or where trauma causes a contusion, or any other surficial or other conditions or imperfections on the skin of a patient or otherwise that benefit from reduced pressure treatment. A wound is thus broadly defined as any damaged region of tissue where fluid may or may not be produced. Examples of such wounds include, but are not limited to, acute wounds, chronic wounds, surgical incisions and other incisions, subacute and dehisced wounds, traumatic wounds, flaps and skin grafts, lacerations, abrasions, contusions, burns, diabetic ulcers, pressure ulcers, stoma, surgical wounds, trauma and venous ulcers or the like. The components of the TNP system described herein can be particularly suited for incisional wounds that exude a small amount of wound exudate.

Some embodiments of the system are designed to operate without the use of an exudate canister. Some embodiments can be configured to support an exudate canister. In some embodiments, configuring the pump assembly 150 and tubing 140 so that the tubing 140 can be quickly and easily removed from the pump assembly 150 can facilitate or improve the process of dressing or pump changes, if necessary. Any of the pump embodiments disclosed herein can be configured to have any suitable connection between the tubing and the pump.

The pump assembly 150 can be configured to deliver negative pressure of approximately -80 mmHg, or between about -20 mmHg and 200 mmHg in some implementations. Note that these pressures are relative to normal ambient atmospheric pressure thus, -200 mmHg would be about 560 mmHg in practical terms. The pressure range can be between about -40 mmHg and -150 mmHg. Alternatively a pressure range of up to - 75 mmHg, up to -80 mmHg or over -80 mmHg can be used. Also a pressure range of below - 75 mmHg can be used. Alternatively a pressure range of over approximately -100 mmHg, or even 150 mmHg, can be supplied by the pump assembly 150.

In operation, the wound filler 130 is inserted into the wound cavity 110 and wound cover 120 is placed so as to seal the wound cavity 110. The pump assembly 150 provides a source of a negative pressure to the wound cover 120, which is transmitted to the wound cavity 110 via the wound filler 130. Fluid (e.g., wound exudate) is drawn through the conduit 140, and can be stored in a canister. In some embodiments, fluid is absorbed by the wound filler 130 or one or more absorbent layers (not shown).

Wound dressings that may be utilized with the pump assembly and other embodiments ofthe present application include Renasys-F, Renasys-G, Renasys AB, and Pico Dressings available from Smith & Nephew. Further description of such wound dressings and other components of a negative pressure wound therapy system that may be used with the pump assembly and other embodiments of the present application are found in U.S. Patent Publication Nos. 2011/0213287, 2011/0282309, 2012/0116334, 2012/0136325, and 2013/0110058.

### Self-contained Wound Dressing

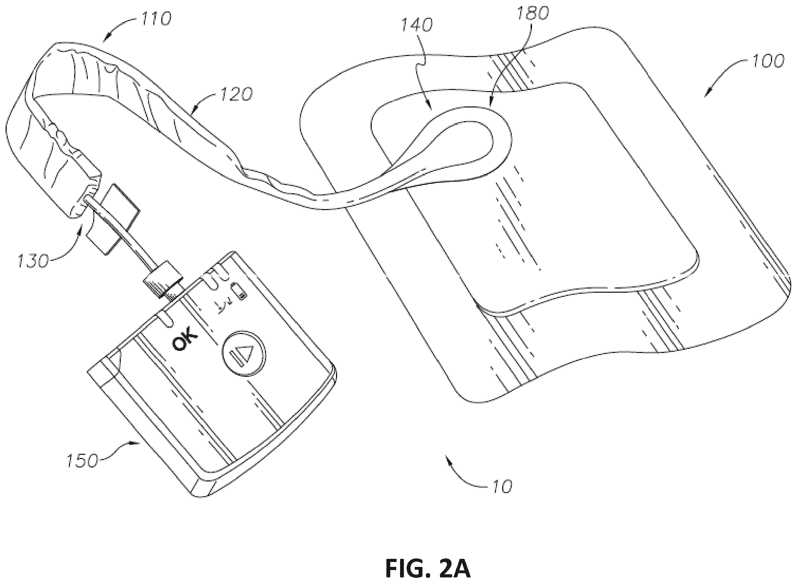

In certain embodiments, NPWT may be applied from a suitable source such as a pump, to a wound through a self-contained wound dressing, such as a PICO^{™} wound dressing, as sold by Smith & Nephew. FIGS. 2A-B illustrate embodiments of a negative pressure wound treatment system 10 employing a wound dressing 100 in conjunction with a fluidic connector 110. Here, the fluidic connector 110 may comprise an elongate conduit, more preferably a bridge 120 having a proximal end 130 and a distal end 140, and an applicator 180 at the distal end 140 of the bridge 120. An optional coupling 160 is preferably disposed at the proximal end 130 of the bridge 120. A cap 170 may be provided with the system (and can in some cases, as illustrated, be attached to the coupling 160). The cap 170 can be useful in preventing fluids from leaking out of the proximal end 130. The system 10 may include a source of negative pressure such as a pump or negative pressure unit 150 capable of supplying negative pressure. The pump may comprise a canister or other container for the storage of wound exudates and other fluids that may be removed from the wound. A canister or container may also be provided separate from the pump. In some embodiments, the pump 150 can be a canisterless pump such as the PICO^{™} pump, as sold by Smith & Nephew. The pump 150 may be connected to the coupling 160 via a tube 190, or the pump 150 may be connected directly to the coupling 160 or directly to the bridge 120. In use, the dressing 100 is placed over a suitably-prepared wound, which may in some cases be filled with a wound packing material such as foam or gauze. The applicator 180 of the fluidic connector 110 has a sealing surface that is placed over an aperture in the dressing 100 and is sealed to the top surface of the dressing 100. Either before, during, or after connection of the fluidic connector 110 to the dressing 100, the pump 150 is connected via the tube 190 to the coupling 160, or is connected directly to the coupling 160 or to the bridge 120. The pump is then activated, thereby supplying negative pressure to the wound. Application of negative pressure may be applied until a desired level of healing of the wound is achieved.

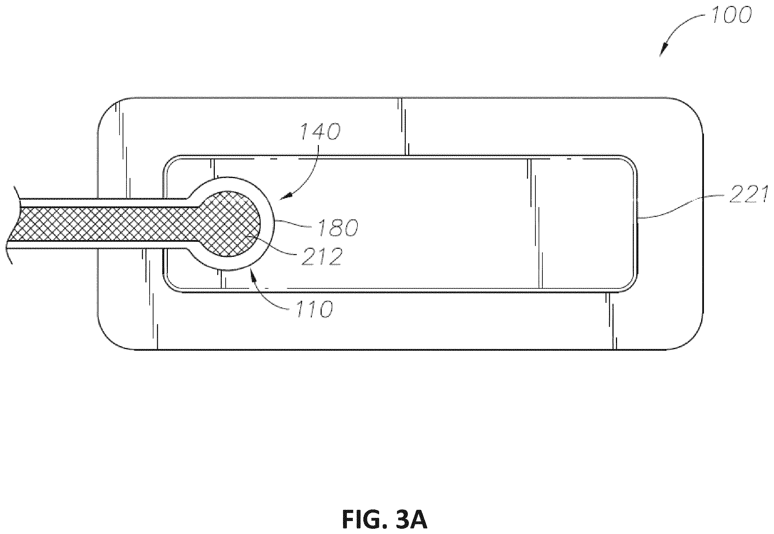

As shown in FIG. 3A, the fluidic connector 110 preferably comprises an enlarged distal end, or head 140 that is in fluidic communication with the dressing 100 as will be described in further detail below. In one embodiment, the enlarged distal end has a round or circular shape. The head 140 is illustrated here as being positioned near an edge of the dressing 100, but may also be positioned at any location on the dressing. For example, some embodiments may provide for a centrally or off-centered location not on or near an edge or corner of the dressing 100. In some embodiments, the dressing 10 may comprise two or more fluidic connectors 110, each comprising one or more heads 140, in fluidic communication therewith. In a preferred embodiment, the head 140 may measure 30mm along its widest edge. The head 140 forms at least in part the applicator 180, described above, that is configured to seal against a top surface of the wound dressing.

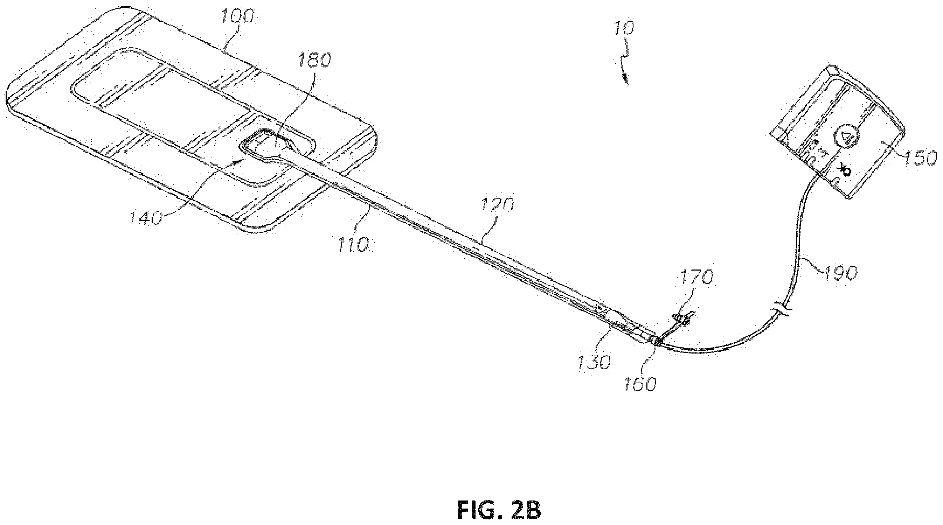

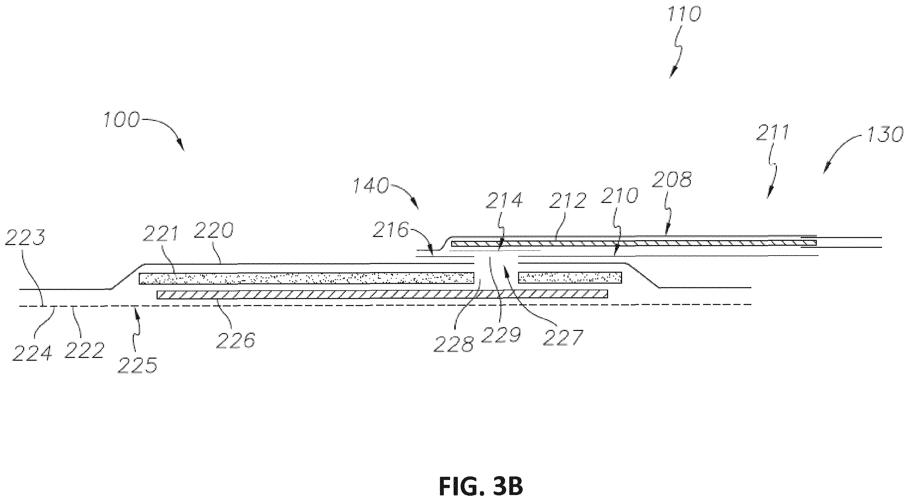

FIG. 3B illustrates a cross-section through a wound dressing 100 similar to the wound dressing 10 as shown in FIG. 2B and described in International Patent Publication WO2013175306 A2, along with fluidic connector 110. The wound dressing 100, which can alternatively be any wound dressing embodiment disclosed herein or any combination of features of any number of wound dressing embodiments disclosed herein, can be located over a wound site to be treated. The dressing 100 may be placed as to form a sealed cavity over the wound site. In a preferred embodiment, the dressing 100 comprises a top or cover layer, or backing layer 220 attached to an optional wound contact layer 222, both of which are described in greater detail below. These two layers 220, 222 are preferably joined or sealed together so as to define an interior space or chamber. This interior space or chamber may comprise additional structures that may be adapted to distribute or transmit negative pressure, store wound exudate and other fluids removed from the wound, and other functions which will be explained in greater detail below. Examples of such structures, described below, include a transmission layer 226 and an absorbent layer 221.

As used herein the upper layer, top layer, or layer above refers to a layer furthest from the surface of the skin or wound while the dressing is in use and positioned over the wound. Accordingly, the lower surface, lower layer, bottom layer, or layer below refers to the layer that is closest to the surface of the skin or wound while the dressing is in use and positioned over the wound.

As illustrated in FIG. 3B, the wound contact layer 222 can be a polyurethane layer or polyethylene layer or other flexible layer which is perforated, for example via a hot pin process, laser ablation process, ultrasound process or in some other way or otherwise made permeable to liquid and gas. The wound contact layer 222 has a lower surface 224 and an upper surface 223. The perforations 225 preferably comprise through holes in the wound contact layer 222 which enable fluid to flow through the layer 222. The wound contact layer 222 helps prevent tissue ingrowth into the other material of the wound dressing. Preferably, the perforations are small enough to meet this requirement while still allowing fluid to flow therethrough. For example, perforations formed as slits or holes having a size ranging from 0.025 mm to 1.2 mm are considered small enough to help prevent tissue ingrowth into the wound dressing while allowing wound exudate to flow into the dressing. In some configurations, the wound contact layer 222 may help maintain the integrity ofthe entire dressing 100 while also creating an air tight seal around the absorbent pad in order to maintain negative pressure at the wound.

Some embodiments of the wound contact layer 222 may also act as a carrier for an optional lower and upper adhesive layer (not shown). For example, a lower pressure sensitive adhesive may be provided on the lower surface 224 of the wound dressing 100 whilst an upper pressure sensitive adhesive layer may be provided on the upper surface 223 of the wound contact layer. The pressure sensitive adhesive, which may be a silicone, hot melt, hydrocolloid or acrylic based adhesive or other such adhesives, may be formed on both sides or optionally on a selected one or none of the sides of the wound contact layer. When a lower pressure sensitive adhesive layer is utilized may be helpful to adhere the wound dressing 100 to the skin around a wound site. In some embodiments, the wound contact layer may comprise perforated polyurethane film. The lower surface of the film may be provided with a silicone pressure sensitive adhesive and the upper surface may be provided with an acrylic pressure sensitive adhesive, which may help the dressing maintain its integrity. In some embodiments, a polyurethane film layer may be provided with an adhesive layer on both its upper surface and lower surface, and all three layers may be perforated together.

A layer 226 of porous material can be located above the wound contact layer 222. This porous layer, or transmission layer, 226 allows transmission of fluid including liquid and gas away from a wound site into upper layers of the wound dressing. In particular, the transmission layer 226 preferably ensures that an open air channel can be maintained to communicate negative pressure over the wound area even when the absorbent layer has absorbed substantial amounts of exudates. The layer 226 should preferably remain open under the typical pressures that will be applied during negative pressure wound therapy as described above, so that the whole wound site sees an equalized negative pressure. The layer 226 may be formed of a material having a three dimensional structure. For example, a knitted or woven spacer fabric (for example Baltex 7970 weft knitted polyester) or a non-woven fabric could be used.

In some embodiments, the transmission layer 226 comprises a 3D polyester spacer fabric layer including a top layer (that is to say, a layer distal from the wound-bed in use) which is a 84/144 textured polyester, and a bottom layer (that is to say, a layer which lies proximate to the wound bed in use) which is a 10 denier flat polyester and a third layer formed sandwiched between these two layers which is a region defined by a knitted polyester viscose, cellulose or the like monofilament fiber. Other materials and other linear mass densities of fiber could of course be used.

Whilst reference is made throughout this disclosure to a monofilament fiber it will be appreciated that a multistrand alternative could of course be utilized. The top spacer fabric thus has more filaments in a yarn used to form it than the number of filaments making up the yarn used to form the bottom spacer fabric layer.

This differential between filament counts in the spaced apart layers helps control moisture flow across the transmission layer. Particularly, by having a filament count greater in the top layer, that is to say, the top layer is made from a yarn having more filaments than the yarn used in the bottom layer, liquid tends to be wicked along the top layer more than the bottom layer. In use, this differential tends to draw liquid away from the wound bed and into a central region of the dressing where the absorbent layer 221 helps lock the liquid away or itself wicks the liquid onwards towards the cover layer where it can be transpired.

Preferably, to improve the liquid flow across the transmission layer 226 (that is to say perpendicular to the channel region formed between the top and bottom spacer layers, the 3D fabric may be treated with a dry cleaning agent (such as, but not limited to, Perchloro Ethylene) to help remove any manufacturing products such as mineral oils, fats and/or waxes used previously which might interfere with the hydrophilic capabilities of the transmission layer. In some embodiments, an additional manufacturing step can subsequently be carried in which the 3D spacer fabric is washed in a hydrophilic agent (such as, but not limited to, Feran Ice 30g/1 available from the Rudolph Group). This process step helps ensure that the surface tension on the materials is so low that liquid such as water can enter the fabric as soon as it contacts the 3D knit fabric. This also aids in controlling the flow of the liquid insult component of any exudates.

A layer 221 of absorbent material is provided above the transmission layer 226. The absorbent material, which comprise a foam or non-woven natural or synthetic material, and which may optionally comprise a super-absorbent material, forms a reservoir for fluid, particularly liquid, removed from the wound site. In some embodiments, the layer 10 may also aid in drawing fluids towards the backing layer 220.

The material of the absorbent layer 221 may also prevent liquid collected in the wound dressing 100 from flowing freely within the dressing, and preferably acts so as to contain any liquid collected within the dressing. The absorbent layer 221 also helps distribute fluid throughout the layer via a wicking action so that fluid is drawn from the wound site and stored throughout the absorbent layer. This helps prevent agglomeration in areas of the absorbent layer. The capacity of the absorbent material must be sufficient to manage the exudates flow rate of a wound when negative pressure is applied. Since in use the absorbent layer experiences negative pressures the material of the absorbent layer is chosen to absorb liquid under such circumstances. A number of materials exist that are able to absorb liquid when under negative pressure, for example superabsorber material. The absorbent layer 221 may typically be manufactured from ALLEVYN^{™} foam, Freudenberg 114-224-4 and/or Chem-Posite^{™}11C-450. In some embodiments, the absorbent layer 221 may comprise a composite comprising superabsorbent powder, fibrous material such as cellulose, and bonding fibers. In a preferred embodiment, the composite is an airlaid, thermally-bonded composite.

In some embodiments, the absorbent layer 221 is a layer of non-woven cellulose fibers having super-absorbent material in the form of dry particles dispersed throughout. Use of the cellulose fibers introduces fast wicking elements which help quickly and evenly distribute liquid taken up by the dressing. The juxtaposition of multiple strand-like fibers leads to strong capillary action in the fibrous pad which helps distribute liquid. In this way, the super-absorbent material is efficiently supplied with liquid. The wicking action also assists in bringing liquid into contact with the upper cover layer to aid increase transpiration rates of the dressing.

An aperture, hole, or orifice 227 is preferably provided in the backing layer 220 to allow a negative pressure to be applied to the dressing 100. The fluidic connector 110 is preferably attached or sealed to the top of the backing layer 220 over the orifice 227 made into the dressing 100, and communicates negative pressure through the orifice 227. A length of tubing may be coupled at a first end to the fluidic connector 110 and at a second end to a pump unit (not shown) to allow fluids to be pumped out of the dressing. Where the fluidic connector is adhered to the top layer of the wound dressing, a length of tubing may be coupled at a first end of the fluidic connector such that the tubing, or conduit, extends away from the fluidic connector parallel or substantially to the top surface of the dressing. The fluidic connector 110 may be adhered and sealed to the backing layer 220 using an adhesive such as an acrylic, cyanoacrylate, epoxy, UV curable or hot melt adhesive. The fluidic connector 110 may be formed from a soft polymer, for example a polyethylene, a polyvinyl chloride, a silicone or polyurethane having a hardness of 30 to 90 on the Shore A scale. In some embodiments, the fluidic connector 110 may be made from a soft or conformable material.

Preferably the absorbent layer 221 includes at least one through hole 228 located so as to underlie the fluidic connector 110. The through hole 228 may in some embodiments be the same size as the opening 227 in the backing layer, or may be bigger or smaller. As illustrated in FIG. 3B a single through hole can be used to produce an opening underlying the fluidic connector 110. It will be appreciated that multiple openings could alternatively be utilized. Additionally should more than one port be utilized according to certain embodiments of the present disclosure one or multiple openings may be made in the absorbent layer and the obscuring layer in registration with each respective fluidic connector. Although not essential to certain embodiments of the present disclosure the use of through holes in the super-absorbent layer may provide a fluid flow pathway which remains unblocked in particular when the absorbent layer is near saturation.

The aperture or through-hole 228 is preferably provided in the absorbent layer 221 beneath the orifice 227 such that the orifice is connected directly to the transmission layer 226 as illustrated in FIG. 3B. This allows the negative pressure applied to the fluidic connector 110 to be communicated to the transmission layer 226 without passing through the absorbent layer 221. This ensures that the negative pressure applied to the wound site is not inhibited by the absorbent layer as it absorbs wound exudates. In other embodiments, no aperture may be provided in the absorbent layer 221, or alternatively a plurality of apertures underlying the orifice 227 may be provided. In further alternative embodiments, additional layers such as another transmission layer or an obscuring layer such as described in International Patent Publication WO2014020440, may be provided over the absorbent layer 221 and beneath the backing layer 220.

The backing layer 220 is preferably gas impermeable, but moisture vapor permeable, and can extend across the width of the wound dressing 100. The backing layer 220, which may for example be a polyurethane film (for example, Elastollan SP9109) having a pressure sensitive adhesive on one side, is impermeable to gas and this layer thus operates to cover the wound and to seal a wound cavity over which the wound dressing is placed. In this way an effective chamber is made between the backing layer 220 and a wound site where a negative pressure can be established. The backing layer 220 is preferably sealed to the wound contact layer 222 in a border region around the circumference of the dressing, ensuring that no air is drawn in through the border area, for example via adhesive or welding techniques. The backing layer 220 protects the wound from external bacterial contamination (bacterial barrier) and allows liquid from wound exudates to be transferred through the layer and evaporated from the film outer surface. The backing layer 220 preferably comprises two layers; a polyurethane film and an adhesive pattern spread onto the film. The polyurethane film is preferably moisture vapor permeable and may be manufactured from a material that has an increased water transmission rate when wet. In some embodiments the moisture vapor permeability of the backing layer increases when the backing layer becomes wet. The moisture vapor permeability of the wet backing layer may be up to about ten times more than the moisture vapor permeability of the dry backing layer.

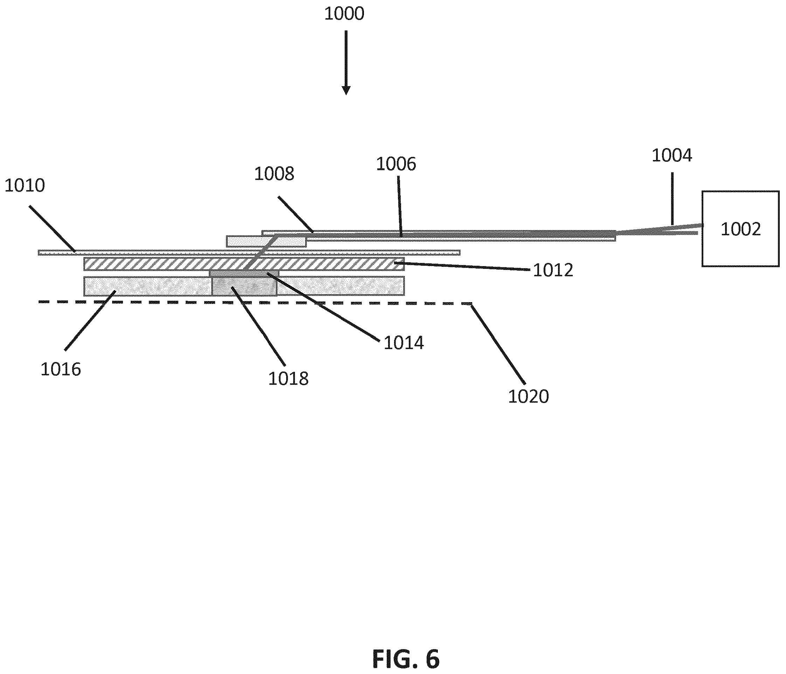

The absorbent layer 221 may be of a greater area than the transmission layer 226, such that the absorbent layer overlaps the edges of the transmission layer 226, thereby ensuring that the transmission layer does not contact the backing layer 220. This provides an outer channel of the absorbent layer 221 that is in direct contact with the wound contact layer 222, which aids more rapid absorption of exudates to the absorbent layer. Furthermore, this outer channel ensures that no liquid is able to pool around the circumference of the wound cavity, which may otherwise seep through the seal around the perimeter of the dressing leading to the formation of leaks. As illustrated in FIGS. 6A-6B, the absorbent layer 221 may define a smaller perimeter than that of the backing layer 220, such that a boundary or border region is defined between the edge of the absorbent layer 221 and the edge of the backing layer 220.

As shown in FIG. 3B, one embodiment of the wound dressing 100 comprises an aperture 228 in the absorbent layer 221 situated underneath the fluidic connector 110. In use, for example when negative pressure is applied to the dressing 100, a wound facing portion of the fluidic connector may thus come into contact with the transmission layer 226, which can thus aid in transmitting negative pressure to the wound site even when the absorbent layer 221 is filled with wound fluids. Some embodiments may have the backing layer 220 be at least partly adhered to the transmission layer 226. In some embodiments, the aperture 228 is at least 1-2 mm larger than the diameter of the wound facing portion of the fluidic connector 11, or the orifice 227.

In particular for embodiments with a single fluidic connector 110 and through hole, it may be preferable for the fluidic connector 110 and through hole to be located in an off-center position as illustrated in FIG. 3A. Such a location may permit the dressing 100 to be positioned onto a patient such that the fluidic connector 110 is raised in relation to the remainder of the dressing 100. So positioned, the fluidic connector 110 and the filter 214 may be less likely to come into contact with wound fluids that could prematurely occlude the filter 214 so as to impair the transmission of negative pressure to the wound site.

Turning now to the fluidic connector 110, preferred embodiments comprise a sealing surface 216, a bridge 211 (corresponding to bridge 120 in FIGS. 2A-2B) with a proximal end 130 and a distal end 140, and a filter 214. The sealing surface 216 preferably forms the applicator previously described that is sealed to the top surface of the wound dressing. In some embodiments a bottom layer of the fluidic connector 110 may comprise the sealing surface 216. The fluidic connector 110 may further comprise an upper surface vertically spaced from the sealing surface 216, which in some embodiments is defined by a separate upper layer of the fluidic connector. In other embodiments the upper surface and the lower surface may be formed from the same piece of material. In some embodiments the sealing surface 216 may comprise at least one aperture 229 therein to communicate with the wound dressing. In some embodiments the filter 214 may be positioned across the opening 229 in the sealing surface, and may span the entire opening 229. The sealing surface 216 may be configured for sealing the fluidic connector to the cover layer of the wound dressing, and may comprise an adhesive or weld. In some embodiments, the sealing surface 216 may be placed over an orifice in the cover layer with optional spacer elements 215 configured to create a gap between the filter 214 and the transmission layer 226. In other embodiments, the sealing surface 216 may be positioned over an orifice in the cover layer and an aperture in the absorbent layer 220, permitting the fluidic connector 110 to provide air flow through the transmission layer 226. In some embodiments, the bridge 211 may comprise a first fluid passage 212 in communication with a source of negative pressure, the first fluid passage 212 comprising a porous material, such as a 3D knitted material, which may be the same or different than the porous layer 226 described previously. The bridge 211 is preferably encapsulated by at least one flexible film layer 208, 210 having a proximal and distal end and configured to surround the first fluid passage 212, the distal end of the flexible film being connected the sealing surface 216. The filter 214 is configured to substantially prevent wound exudate from entering the bridge, and spacer elements 215 are configured to prevent the fluidic connector from contacting the transmission layer 226. These elements will be described in greater detail below.

Some embodiments may further comprise an optional second fluid passage positioned above the first fluid passage 212. For example, some embodiments may provide for an air leak may be disposed at the proximal end of the top layer that is configured to provide an air path into the first fluid passage 212 and dressing 100 similar to the suction adapter as described in U.S. Patent No 8,801,685.

Preferably, the fluid passage 212 is constructed from a compliant material that is flexible and that also permits fluid to pass through it if the spacer is kinked or folded over. Suitable materials for the fluid passage 212 include without limitation foams, including open-cell foams such as polyethylene or polyurethane foam, meshes, 3D knitted fabrics, non-woven materials, and fluid channels. In some embodiments, the fluid passage 212 may be constructed from materials similar to those described above in relation to the transmission layer 226. Advantageously, such materials used in the fluid passage 212 not only permit greater patient comfort, but may also provide greater kink resistance, such that the fluid passage 212 is still able to transfer fluid from the wound toward the source of negative pressure while being kinked or bent.

In some embodiments, the fluid passage 212 may be comprised of a wicking fabric, for example a knitted or woven spacer fabric (such as a knitted polyester 3D fabric, Baltex 7970^{®}, or Gehring 879^{®}) or a nonwoven fabric. These materials selected are preferably suited to channeling wound exudate away from the wound and for transmitting negative pressure and/or vented air to the wound site, and may also confer a degree of kinking or occlusion resistance to the fluid passage 212. In some embodiments, the wicking fabric may have a three-dimensional structure, which in some cases may aid in wicking fluid or transmitting negative pressure. In certain embodiments, including wicking fabrics, these materials remain open and capable of communicating negative pressure to a wound area under the typical pressures used in negative pressure therapy, for example between 40 to 150 mmHg. In some embodiments, the wicking fabric may comprise several layers of material stacked or layered over each other, which may in some cases be useful in preventing the fluid passage 212 from collapsing under the application of negative pressure. In other embodiments, the wicking fabric used in the fluid passage 212 may be between 1.5 mm and 6 mm; more preferably, the wicking fabric may be between 3 mm and 6 mm thick, and may be comprised of either one or several individual layers of wicking fabric. In other embodiments, the fluid passage 212 may be between 1.2-3 mm thick, and preferably thicker than 1.5 mm. Some embodiments, for example a suction adapter used with a dressing which retains liquid such as wound exudate, may employ hydrophobic layers in the fluid passage 212, and only gases may travel through the fluid passage 212. Additionally, and as described previously, the materials used in the system are preferably conformable and soft, which may help to avoid pressure ulcers and other complications which may result from a wound treatment system being pressed against the skin of a patient.

Preferably, the filter element 214 is impermeable to liquids, but permeable to gases, and is provided to act as a liquid barrier and to ensure that no liquids are able to escape from the wound dressing 100. The filter element 214 may also function as a bacterial barrier. Typically the pore size is 0.2µm. Suitable materials for the filter material of the filter element 214 include 0.2 micron Gore^{™} expanded PTFE from the MMT range, PALL Versapore^{™} 200R, and Donaldson^{™} TX6628. Larger pore sizes can also be used but these may require a secondary filter layer to ensure full bioburden containment. As wound fluid contains lipids it is preferable, though not essential, to use an oleophobic filter membrane for example 1.0 micron MMT-332 prior to 0.2 micron MMT-323. This prevents the lipids from blocking the hydrophobic filter. The filter element can be attached or sealed to the port and/or the cover film over the orifice. For example, the filter element 214 may be molded into the fluidic connector 110, or may be adhered to one or both of the top of the cover layer and bottom of the suction adapter 110 using an adhesive such as, but not limited to, a UV cured adhesive.

It will be understood that other types of material could be used for the filter element 214. More generally a microporous membrane can be used which is a thin, flat sheet of polymeric material, this contains billions of microscopic pores. Depending upon the membrane chosen these pores can range in size from 0.01 to more than 10 micrometers. Microporous membranes are available in both hydrophilic (water filtering) and hydrophobic (water repellent) forms. In some embodiments of the present disclosure, filter element 214 comprises a support layer and an acrylic co-polymer membrane formed on the support layer. Preferably the wound dressing 100 according to certain embodiments of the present disclosure uses microporous hydrophobic membranes (MHMs). Numerous polymers may be employed to form MHMs. For example, the MHMs may be formed from one or more of PTFE, polypropylene, PVDF and acrylic copolymer. All of these optional polymers can be treated in order to obtain specific surface characteristics that can be both hydrophobic and oleophobic. As such these will repel liquids with low surface tensions such as multi-vitamin infusions, lipids, surfactants, oils and organic solvents.

MHMs block liquids whilst allowing air to flow through the membranes. They are also highly efficient air filters eliminating potentially infectious aerosols and particles. A single piece of MHM is well known as an option to replace mechanical valves or vents. Incorporation of MHMs can thus reduce product assembly costs improving profits and costs/benefit ratio to a patient.

The filter element 214 may also include an odor absorbent material, for example activated charcoal, carbon fiber cloth or Vitec Carbotec-RT Q2003073 foam, or the like. For example, an odor absorbent material may form a layer of the filter element 214 or may be sandwiched between microporous hydrophobic membranes within the filter element. The filter element 214 thus enables gas to be exhausted through the orifice. Liquid, particulates and pathogens however are contained in the dressing.

The wound dressing 100 may comprise spacer elements 215 in conjunction with the fluidic connector 110 and the filter 214. With the addition of such spacer elements 215 the fluidic connector 110 and filter 214 may be supported out of direct contact with the absorbent layer 220 and/or the transmission layer 226. The absorbent layer 220 may also act as an additional spacer element to keep the filter 214 from contacting the transmission layer 226. Accordingly, with such a configuration contact of the filter 214 with the transmission layer 226 and wound fluids during use may thus be minimized.

Similar to the embodiments of wound dressings described above, some wound dressings comprise a perforated wound contact layer with silicone adhesive on the skin-contact face and acrylic adhesive on the reverse. Above this bordered layer sits a transmission layer or a 3D spacer fabric pad. Above the transmission layer, sits an absorbent layer. The absorbent layer can include a superabsorbent non-woven (NW) pad. The absorbent layer can over-border the transmission layer by approximately 5mm at the perimeter. The absorbent layer can have an aperture or through-hole toward one end. The aperture can be about 10 mm in diameter. Over the transmission layer and absorbent layer lies a backing layer. The backing layer can be a high moisture vapor transmission rate (MVTR) film, pattern coated with acrylic adhesive. The high MVTR film and wound contact layer encapsulate the transmission layer and absorbent layer, creating a perimeter border of approximately 20 mm. The backing layer can have a 10 mm aperture that overlies the aperture in the absorbent layer. A fluidic connector can be bonded above the hole, the fluid connector comprising a liquid-impermeable, gas-permeable semi-permeable membrane (SPM) or filter that overlies the aforementioned apertures.

### Treatment of Abdominal Wounds

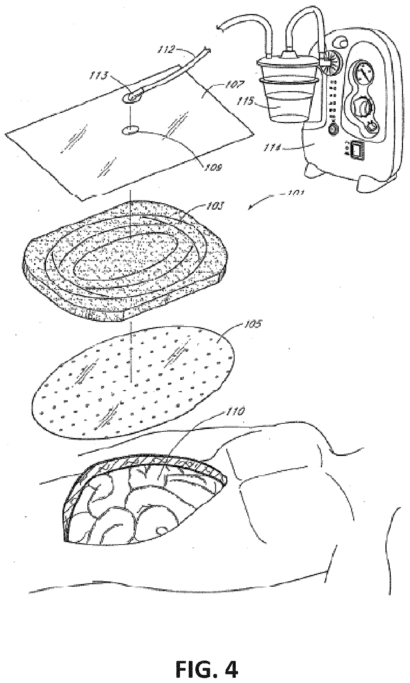

Turning to FIG. 4, treatment of other wound types, such as larger abdominal wounds, with negative pressure in certain embodiments uses a negative pressure treatment system 101 as illustrated schematically here. In this embodiment, a wound site 106, illustrated here as an abdominal wound site, may benefit from treatment with negative pressure. Such abdominal wound sites may be a result of, for example, an accident or due to surgical intervention. In some cases, medical conditions such as abdominal compartment syndrome, abdominal hypertension, sepsis, or fluid edema may require decompression of the abdomen with a surgical incision through the abdominal wall to expose the peritoneal space, after which the opening may need to be maintained in an open, accessible state until the condition resolves. Other conditions may also necessitate that an opening-particularly in the abdominal cavity-remain open, for example if multiple surgical procedures are required (possibly incidental to trauma), or there is evidence of clinical conditions such as peritonitis or necrotizing fasciitis.

In cases where there is a wound, particularly in the abdomen, management of possible complications relating to the exposure of organs and the peritoneal space is desired, whether or not the wound is to remain open or if it will be closed. Therapy, preferably using the application of negative pressure, can be targeted to minimize the risk of infection, while promoting tissue viability and the removal of deleterious substances from the wound site. The application of reduced or negative pressure to a wound site has been found to generally promote faster healing, increased blood flow, decreased bacterial burden, increased rate of granulation tissue formation, to stimulate the proliferation of fibroblasts, stimulate the proliferation of endothelial cells, close chronic open wounds, inhibit burn penetration, and/or enhance flap and graft attachment, among other things. It has also been reported that wounds that have exhibited positive response to treatment by the application of negative pressure include infected open wounds, decubitus ulcers, dehisced incisions, partial thickness burns, and various lesions to which flaps or grafts have been attached. Consequently, the application of negative pressure to a wound site106 can be beneficial to a patient.

Accordingly, certain embodiments provide for a wound contact layer 105 to be placed over the wound site 106. The wound contact layer can also be referred to as an organ protection layer and/or a tissue protection layer. Preferably, the wound contact layer 105 can be a thin, flexible material which will not adhere to the wound site or the exposed viscera in close proximity. For example, polymers such as polyurethane, polyethylene, polytetrafluoroethylene, or blends thereof may be used. In one embodiment, the wound contact layer is permeable. For example, the wound contact layer 105 can be provided with openings, such as holes, slits, or channels, to allow the removal of fluids from the wound site 106 or the transmittal of negative pressure to the wound site 106. Additional embodiments of the wound contact layer 105 are described in further detail below.

Certain embodiments of the negative pressure treatment system 101 may also use a porous wound filler 103, which can be disposed over the wound contact layer 105. This pad 103 can be constructed from a porous material, for example foam, that is soft, resiliently flexible, and generally conformable to the wound site 106. Such a foam can include an open-celled and reticulated foam made, for example, of a polymer. Suitable foams include foams composed of, for example, polyurethane, silicone, and polyvinyl alcohol. Preferably, this pad 103 can channel wound exudate and other fluids through itself when negative pressure is applied to the wound. Some pads 103 may include preformed channels or openings for such purposes. In certain embodiments, the pad 103 may have a thickness between about 2.5 cm and 5.1 cm (one inch and two inches). The pad may also have a length of between about 40.6 cm and 43.2 cm (16 and 17 inches), and a width of between about 27.9 cm and 30.5 cm (11 and 12 inches). In other embodiments, the thickness, width, and/ or length can have other suitable values. Other embodiments of wound fillers that may be used in place of or in addition to the pad 103 are discussed in further detail below.

Preferably, a drape 107 is used to seal the wound site 106. The drape 107 can be at least partially liquid impermeable, such that at least a partial negative pressure may be maintained at the wound site. Suitable materials for the drape 107 include, without limitation, synthetic polymeric materials that do not significantly absorb aqueous fluids, including polyolefins such as polyethylene and polypropylene, polyurethanes, polysiloxanes, polyamides, polyesters, and other copolymers and mixtures thereof. The materials used in the drape may be hydrophobic or hydrophilic. Examples ofsuitable materials include Transeal^{®} available from DeRoyal and OpSite^{®} available from Smith & Nephew. In order to aid patient comfort and avoid skin maceration, the drapes in certain embodiments are at least partly breathable, such that water vapor is able to pass through without remaining trapped under the dressing. An adhesive layer may be provided on at least a portion the underside of the drape 107 to secure the drape to the skin of the patient, although certain embodiments may instead use a separate adhesive or adhesive strip. Optionally, a release layer may be disposed over the adhesive layer to protect it prior to use and to facilitate handling the drape 107; in some embodiments, the release layer may be composed of multiple sections.

The negative pressure system 101 can be connected to a source of negative pressure, for example a pump 114. One example of a suitable pump is the Renasys EZ pump available from Smith & Nephew. The drape 107 may be connected to the source of negative pressure 114 via a conduit 112. The conduit 112 may be connected to a port 113 situated over an aperture 109 in the drape 107, or else the conduit 112 may be connected directly through the aperture 109 without the use of a port. In a further alternative, the conduit may pass underneath the drape and extend from a side of the drape. U.S. Pat. No. 7,524,315 discloses other similar aspects of negative pressure systems.

In many applications, a container or other storage unit 115 may be interposed between the source of negative pressure 114 and the conduit 112 so as to permit wound exudate and other fluids removed from the wound site to be stored without entering the source of negative pressure. Certain types of negative pressure sources-for example, peristaltic pumps-may also permit a container 115 to be placed after the pump 114. Some embodiments may also use a filter to prevent fluids, aerosols, and other microbial contaminants from leaving the container 115 and/or entering the source of negative pressure 114. Further embodiments may also include a shut-off valve or occluding hydrophobic and/or oleophobic filter in the container to prevent overflow; other embodiments may include sensing means, such as capacitive sensors or other fluid level detectors that act to stop or shut off the source of negative pressure should the level of fluid in the container be nearing capacity. At the pump exhaust, it may also be preferable to provide an odor filter, such as an activated charcoal canister.

### Therapeutic Ultrasound Wound Treatment Apparatuses

Throughout the specification and in particular within the paragraphs below, reference may be made to ultrasound (US), ultrasonic energy, ultrasound energy, and/or high frequency vibrational energy. One of skill in the art will understand that high frequency vibrational energy is often used interchangeably with ultrasound or ultrasonic energy by those of skill in the art.

Cellular behavior within multi-cellular organisms is dictated by interactions with the extracellular matrix, the materials and structures outside of a cell that make a grouping of cells into a tissue. Cellular engagement with the extracellular matrix can result in a number of consequences, such as regulation of cell migration and proliferation, secretion, and differentiation. The interaction of cells with the surrounding extracellular matrix is a critical component of wound healing. It is thought that selective application of ultrasound to a wound or other tissue can alter cellular behavior, potentially improving and speeding up the healing process. Fibroblasts are highly prevalent in the human dermis and play a critical role in wound healing. Fibroblasts assist in wound healing by forming granulation tissue and generating new extra-cellular matrix components, such as the formation of collagen at a wound site. However, like many cells, fibroblasts can be impaired by various disease states such as diabetes and other patient comorbidities. Under certain stressful conditions, fibroblasts may develop a stress-induced premature senescence phenotype. In chronic wounds, populations of greater than 15% senescent fibroblasts has been described as a threshold beyond which healing is impaired.

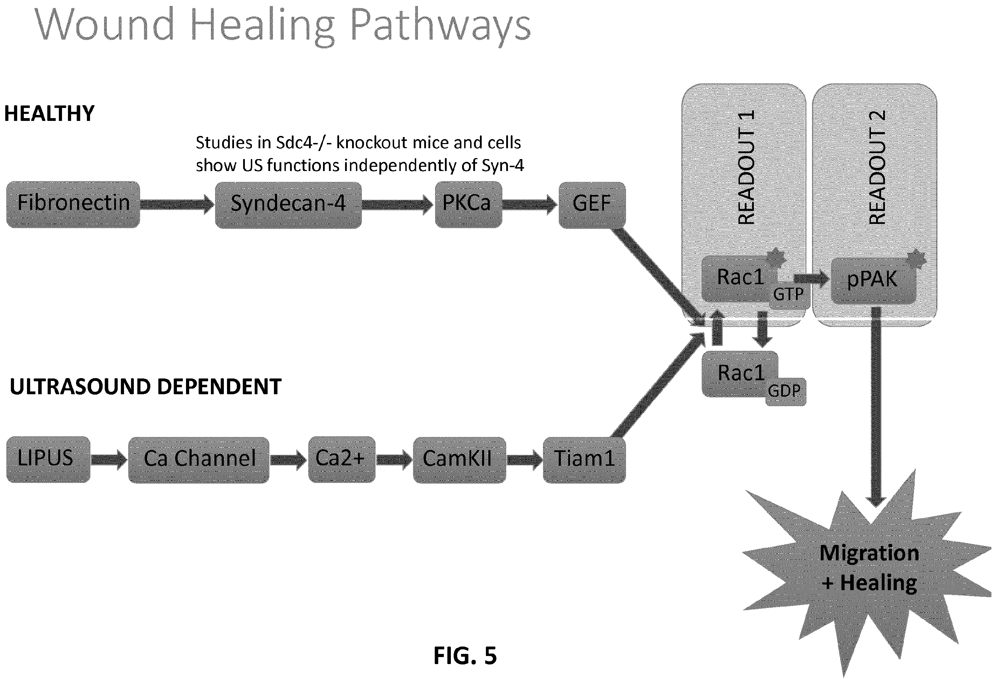

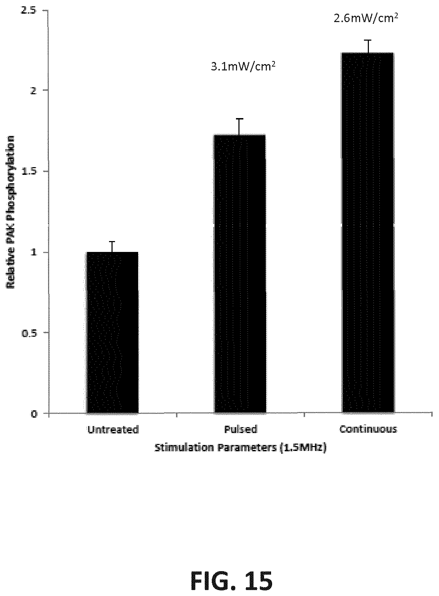

Figure 5 depicts an embodiment of a possible mechanism by which ultrasound may activate an alternative calcium channel dependent pathway resulting in increased Rac1 signaling. Rac1 is a common signaling protein found in human cells, known to increase cellular events related to healing such as glucose uptake, cell growth, cytoskeletal reorganization, antimicrobial cytotoxicity, and the activation of protein kinases. As shown in Figure 5, Rac1 interacts with serine/threonine-protein kinase (PAK) in a Guanosine-5'-triphosphate (GTP) mediated interaction to induce migration and healing. In brief, in healthy fibroblasts, a fibronectin-mediated pathway results in increased cell migration and healing. When this pathway is disrupted via stressors, such as described above, then cellular migration and healing are impaired. However, in instances where the fibronectin-medicated pathway is disrupted, an alternative ultrasound-dependent healing pathway may be activated. By stimulating this ultrasound-dependent pathway, it is thought that therapeutic ultrasound improves wound healing. Further details regarding the signal parameters for optimizing the therapeutic ultrasound signal for maximal wound healing are detailed later in the specification, particularly in Figures 15-21.

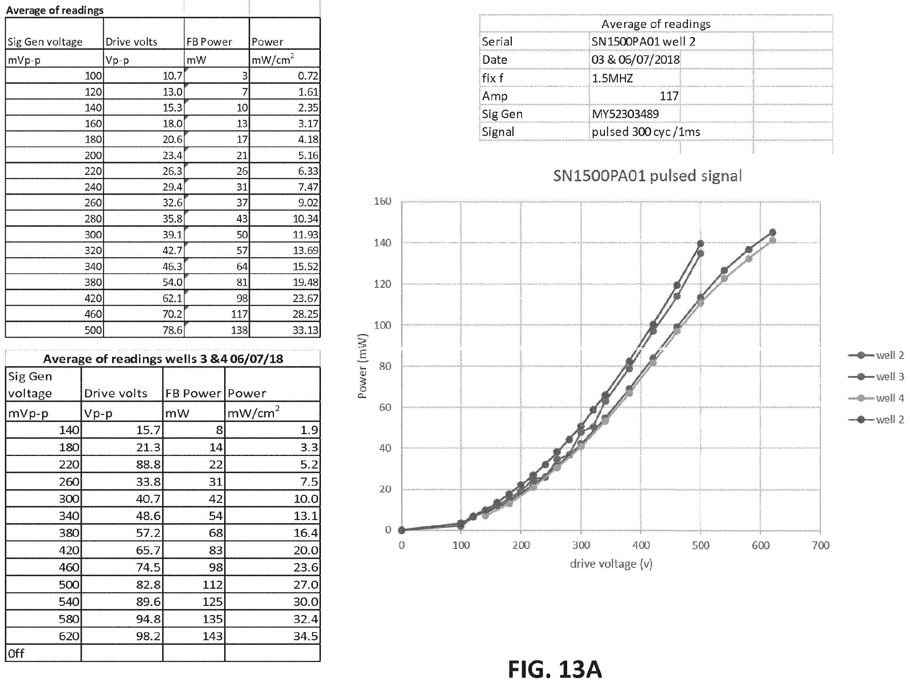

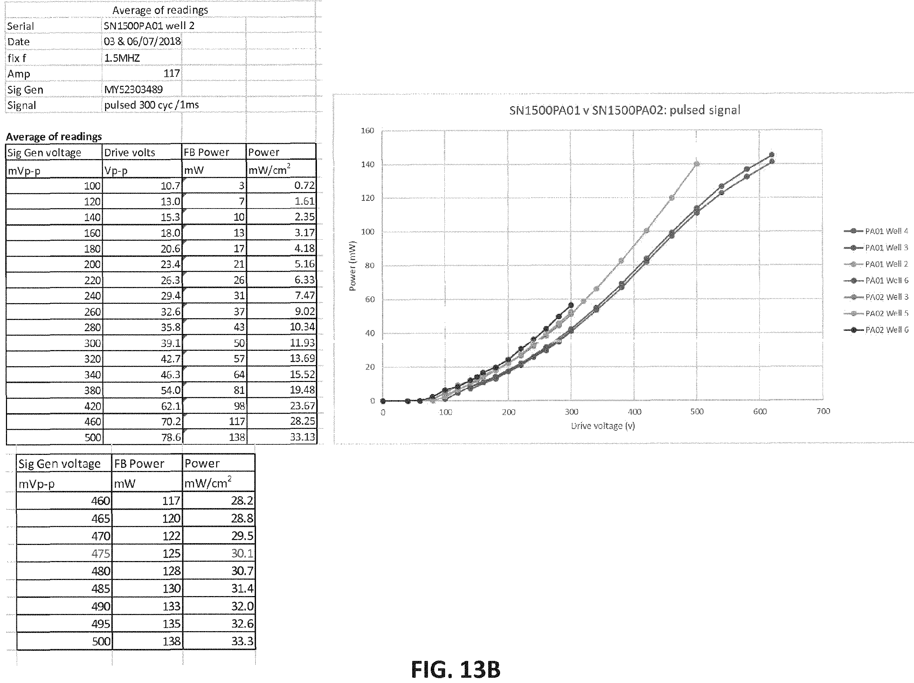

Figure 6 depicts an embodiment of a therapeutic ultrasound wound treatment apparatus 1000 for use in treating a wound, similar to the dressings described above in Figures 1-3B. In certain embodiments, a suitable signal may be delivered to the dressing from an ultrasonic frequency electrical signal generator 1002, via wires 1004. One of skill in the art will understand that the signal may have any parameters such as timing, intensity, and frequency disclosed herein this section or elsewhere in the specification, particularly the parameters disclosed later in the specification with respect to Figures 13-17. The ultrasonic frequency electrical signal generator may be of any suitable type, for example, the EXOGEN generator manufactured by Bioventus. The wires may be contained within a channel 1006 contained within a port 1008, such as the soft port described above in relation to Figures 1-3B. In certain embodiments, the channel 1006 may have 2 layers of spacer to provide space for the wires to pass. The port may be connected to a cover layer 1010, which may be adhered to the port via a constructional adhesive. The cover layer can overlay an absorbent layer 1012, the absorbent layer constructed from any material disclosed herein this section or elsewhere in the specification, for example a cellulose material with embedded superabsorbent particles. An ultrasonic transducer 1014 may be located beneath the absorbent layer. The ultrasonic transducer may be of any suitable type, such as a piezoelectric transducer, a capacitive transducer, and/or any suitable transducer such as those described in PCT. Pub. No. WO1999/056829, PCT. Pub. No. WO 1999/048621, and U.S. Pat. No. 5,904,659. Once the ultrasonic transducer is stimulated by a suitable electrical signal from the signal generator 1002, the transducer will emit a therapeutic ultrasonic signal. The therapeutic signal provided by the transducer may be highly variable in terms of timing of pulsation, frequency, and intensity. Further details regarding the therapeutic signal are provided below. In some embodiments, the therapeutic ultrasound wound treatment apparatuses disclosed herein may comprise about: one, two, three, four, five, ten, fifteen, 25, 50, 75, 100, 150, 200, 300 or more ultrasonic transducers. In certain embodiments, the ultrasonic transducers may be organized into a grid, such as a grid contained within a flexible substrate.

In certain embodiments, the ultrasound signal should be delivered to the wounded surface with sufficient intensity to stimulate healing. Therefore, it is desirable that a delivery pathway be available for the therapeutic ultrasound to be effectively transmitted to the surface. Such a delivery pathway may be provided by a delivery layer 1016 comprising a transmission portion 1018. In certain embodiments, the delivery layer may comprise foam surrounding the transmission portion 1018. For example, the delivery layer may be constructed from a polyurethane foam or any suitable material such as disclosed herein this section or elsewhere in the specification. In some embodiments, the delivery layer may comprise one, two, three, four, five, ten, fifteen, or more transmission portions. The transmission portions may have any suitable shape, such as a column, a pillar, a cuboid, or a rectangular parallelepiped. In embodiments the transmission portion may be in the form of a strip or a series of layered strips adjacent to a transducer. Any shape may be suitable for the transmission portion, provided that there is a continuous transmission portion "line-of-sight" between the transducer and the wounded tissue. Ultrasound transmission is sensitive to the medium used for transmission, therefore without a path to the wound of transmission material, the ultrasound may not reach the wound.

The transmission portion 2018 may be constructed from any suitable material for conveying high frequency vibrational energy, such as a silicone gel, a silicone adhesive (for example 2111 silicone adhesive), Cica Care silicone gel, Durafiber that may be wetted out, or ultrasound connection gel surrounded by a film bubble. In certain embodiments, a series of silicone strands may be embedded in the delivery layer, thereby allowing for multiple ultrasound pathways.

A wound contact layer may be positioned beneath the delivery layer. The wound contact layer may be constructed from any suitable material such as disclosed herein this section or elsewhere in the specification. For example, the wound contact layer may be constructed from polyurethane alone or polyurethane coated with a silicone adhesive on the bottom, top, or both the bottom or the top of the wound contact layer. In some embodiments, the wound contact layer itself may be replaced with a silicone adhesive layer. The wound contact layer may be constructed as a film layer coated in an acrylic constructional adhesive.

One of skill in the art will understand that in embodiments the wound contact layer and the delivery layer, outside of the transmission portion, may be porous thereby allowing wound exudate to pass through the wound contact layer and the delivery layer, to be absorbed within the absorbent layer. In some embodiments, negative pressure may also be applied to the wound treatment apparatuses of Figures 6 -11, thereby allowing for the simultaneous application of therapeutic ultrasound and NPWT. In embodiments, NPWT may be applied in an alternating fashion with therapeutic ultrasound. Advantageously, application of negative pressure may serve to draw the dressing and/or substrate downward toward the wound, thereby bringing the transducers and/or transmission medium(s) directly into contact with tissue and/or exudate. Bringing the dressing or substrate into direct contact with tissue and/or exudate advantageously may reduce signal lost through transmission through air.

One of skill in the art will further understand that the positioning of the various layers and components as described in Figure 6 is for illustrative purposes, and the ultrasound transducer may be positioned in various locations within the dressing. For example, the ultrasound transducer may be placed directly against the wound contact layer, on the sides of the dressing, near the center of the dressing, off-center within the dressing, or any other suitable position.

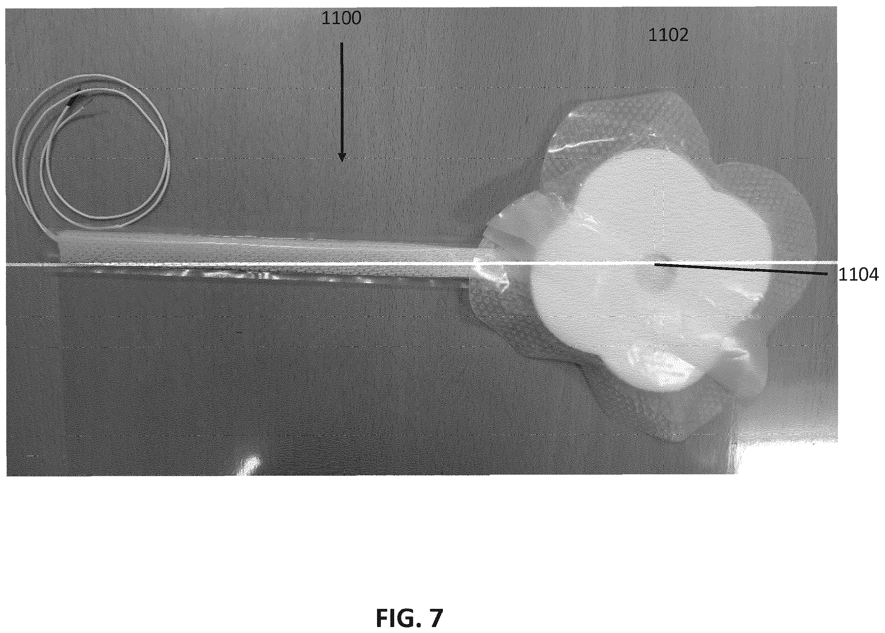

Figure 7 depicts a bottom view of an embodiment of a lobed therapeutic ultrasound wound treatment apparatus 1100, similar to the apparatus of Figure 6. Here the shape of the apparatus may be in the form of a four-lobed dressing 1102. However, in certain embodiments, the dressing may be oval shaped, rectangular, single-lobed, double-lobed, triple-lobed, or comprise five or more lobes. The dressing may further be shaped according to any shape or size disclosed herein this section or elsewhere in the specification. Here, as in Figure 6, the transmission portion 1104 may be centrally located within the dressing portion of the apparatus.

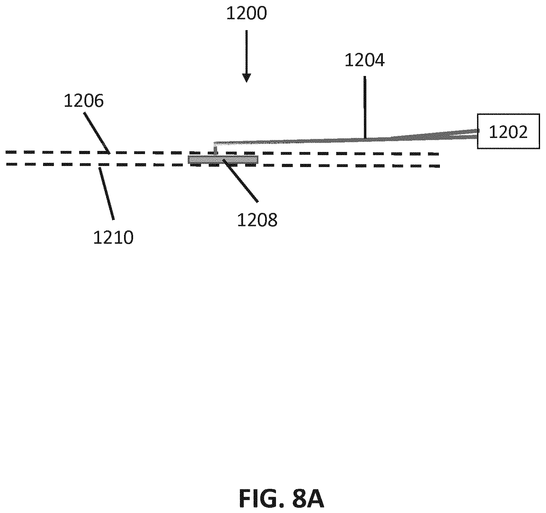

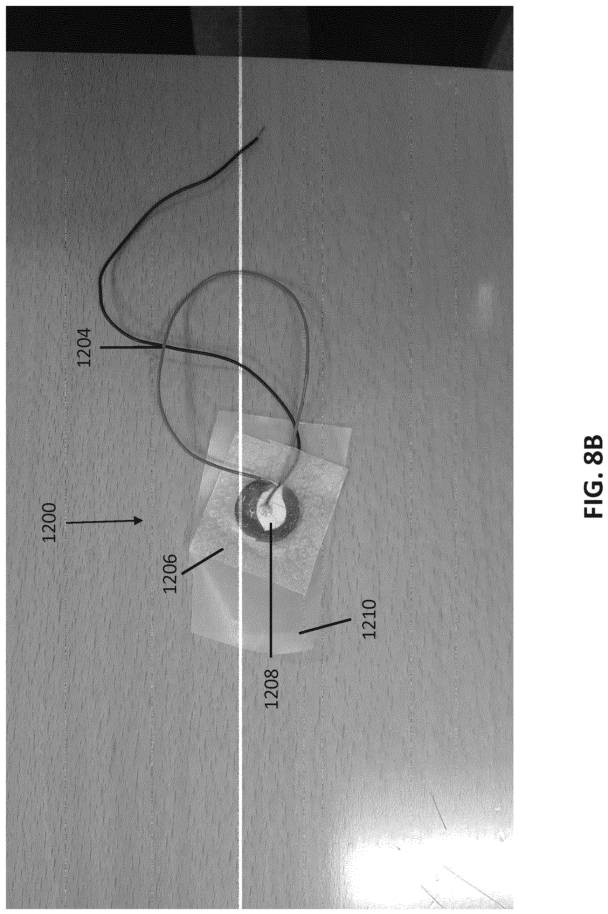

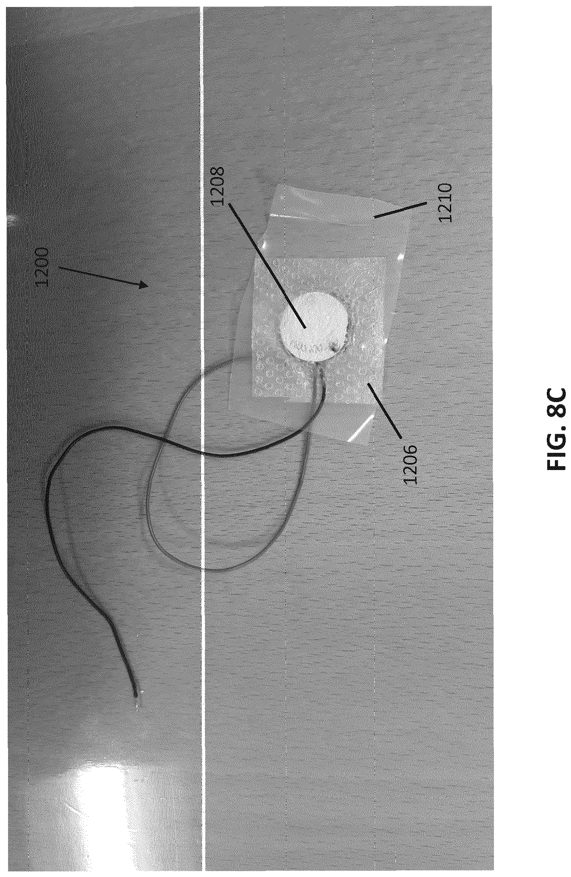

Figure 8A depicts an embodiment of a therapeutic ultrasound wound treatment apparatus 1200 with some similarities to the embodiments of Figures 6 and 7. An ultrasonic frequency electrical signal generator 1202 connects to a transducer 1208 via wires 1204. However, here the transducer 1208 may be placed directly over the wound contact layer 1120. In embodiments, the wound contact member may be any material disclosed herein, such as silicone. The transducer may then be encapsulated by cover layer 1206. Figure 8B is a photograph of the embodiment of Figure 8A, shown from the top, while Figure 8C is a photograph of the embodiment of Figure 8A, shown from the bottom. Wires 1204, cover layer 1206, transducer 1208, and wound contact layer 1210 are shown.

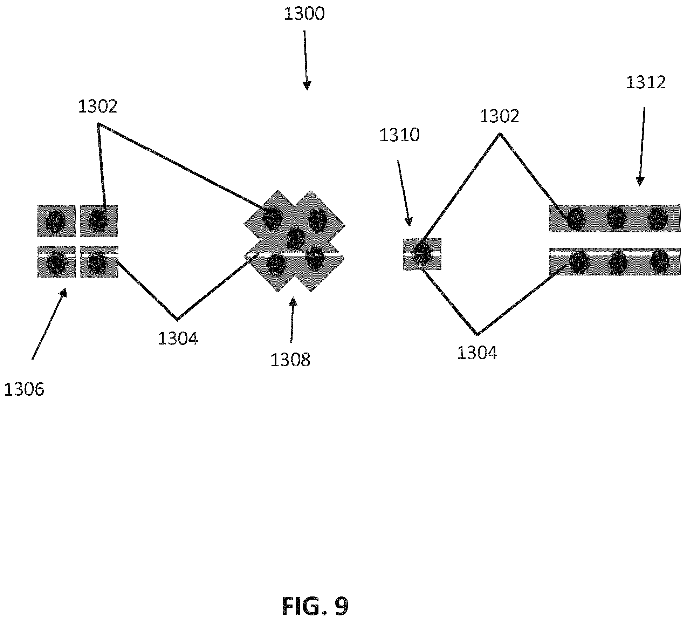

Figure 9 depicts various embodiments of arrangements 1300 oftransducers 1302 and transmission materials 1304 in strip form, such as within an ultrasound wound treatment apparatus as depicted in Figures 7-8C. For example, the transducers and transmission materials can be organized into a square formation with 4 transducers 1306. Alternatively, the transducers can be organized in a cross formation, with 5 transducers 1308. In embodiments, there may be only a single transducer 1310 or rows of transducers 1312 positioned on a single strip or multiple strips of transmission material. In embodiments, the ultrasonic transducers may be arranged in a circular pattern, a spiral pattern, an array, or any suitable arrangement.

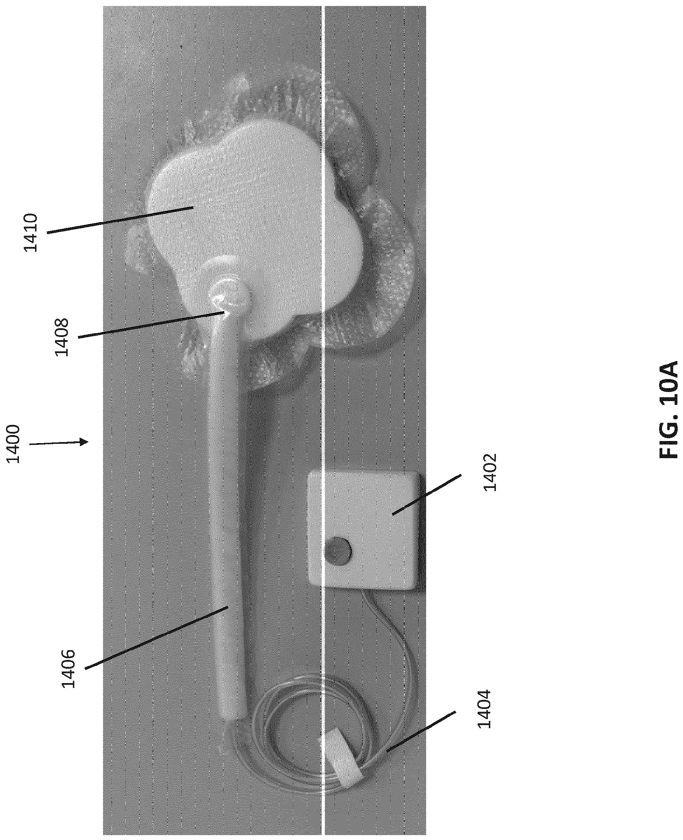

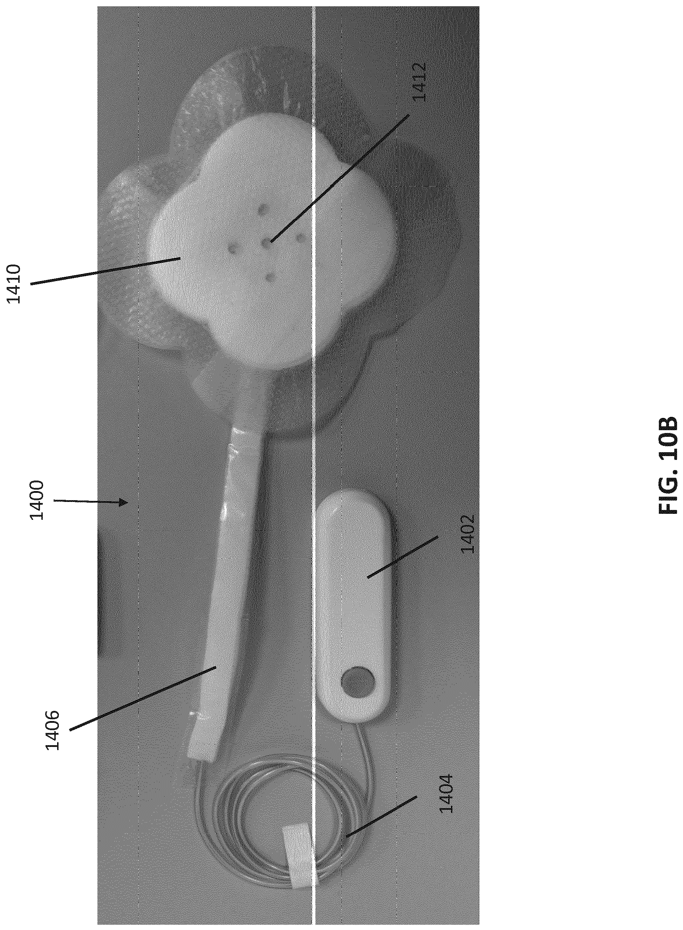

Figures 10A-10B depict an embodiment of a therapeutic ultrasound delivery apparatus 1400 that may be configured to deliver ultrasound alone or deliver both ultrasound and negative pressure. Treatment module 1402 may be an ultrasonic frequency electrical signal generator or a negative pressure generating pump, or contain both an ultrasonic frequency electrical signal generator and a pump. Both wires and tubing 1404 may extend from treatment module 1402 through channel 1406 to port 1408 and absorbent dressing 1410. Dressing 1410 may comprise any construction described herein this section or elsewhere in the specification, particularly as relate to Figures 2A-3B. On the underside of dressing 1410, there may be a plurality of transmission portions 1412, for example five, and a plurality of ultrasonic transducers positioned within the dressing (not shown). The number of transmission portions and ultrasonic transducers may be of any number disclosed herein this section or elsewhere in the specification.

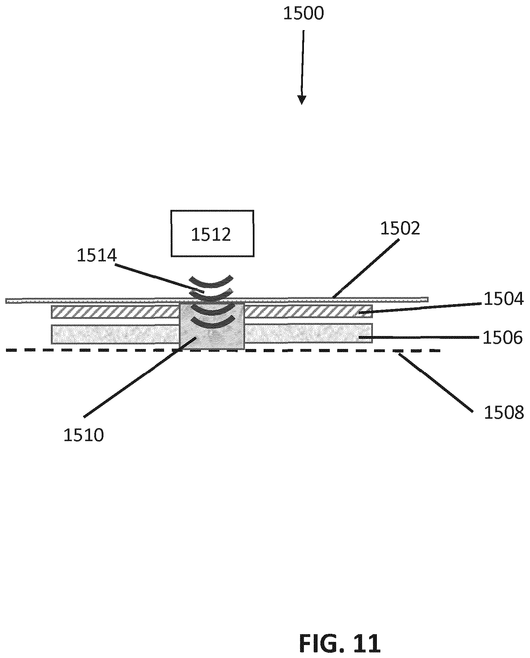

Figure 11 depicts an embodiment of a therapeutic ultrasound delivery apparatus 1500 similar to the apparatuses of Figures 6, 7, 10A, and 10B and the dressings described above in relation to Figures 2-3. Here, the apparatus includes a cover layer 1502, an absorbent layer 1504, a delivery lay 1506, and a wound contact layer 1508. However, a transmission portion extends from the wound contact layer through the delivery layer and the absorbent layer to the cover layer 1502. The positioning of the transmission portion now allows an ultrasound pathway from the top of the dressing to the bottom of the dressing. Therefore an ultrasonic transducer 1512 may be positioned over the transmission portion 1510 and deliver therapeutic ultrasound through the dressing 1500. In some embodiments, the transducer may be mounted to the top of the dressing or may be an external device that can be applied and removed at will.

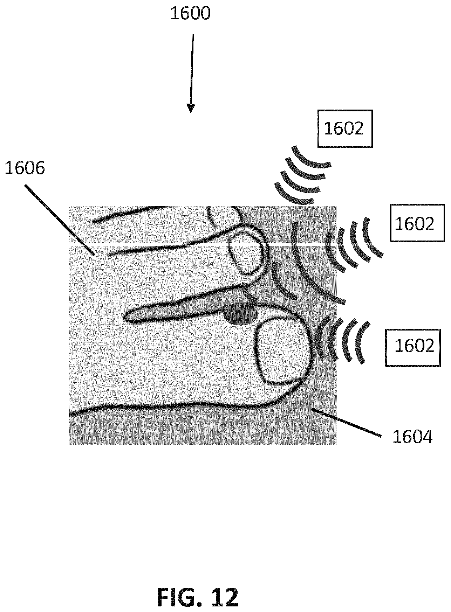

Figure 12 depicts an embodiment of a diabetic foot ulcer treatment bath 1600. A plurality of ultrasonic transducers 1602 may be positioned around a container filled with liquid 1604, configured to receive a human foot 1606. Since liquid acts as a medium for transmission of ultrasound 1606, ultrasound may be delivered to the foot from multiple angles from the plurality of transducers. Due to the contours of the foot, it is particularly difficult to treat foot wounds, therefore by creating a bath of ultrasound, it is possible to ensure that even the most difficult to reach wound areas are bathed in ultrasound.

### Therapeutic Ultrasound Signal

As described above, fibroblasts may be sensitive to application of ultrasound, increasing migration and healing in response to ultrasound signal delivery within a wound. However, the parameters of an ultrasound signal can be modified in a number of ways, such as by altering the voltage, frequency, or other parameters.

In certain embodiments, the frequency of the therapeutic ultrasound signal may range from: about .1 - 10MHz, about .5-9MHz, about 1-8MHz, about 1.5 - 7MHz, about 2-6MHz, 3-5MHz, or about 4MHz. In some embodiments the frequency may be about .5MHz, about 1MHz, about 1.5MHz, about 2MHz, about 2.5MHz, about 3MHz, about 3.5MHz, about 4.0MHz, about 4.5MHz, about 5MHz, or greater than about 5MHz.

In some embodiments, the acoustic power (power per unit area) of the therapeutic ultrasound signal may range from about .1mW/cm² to 500mW/cm², about 1 mW/cm² to 400mW/cm², about 10mW/cm² to 300mW/cm², about 20mW/cm² to 200mW/cm², 30 to 100 mW/cm², or about 40-50 mW/cm². The acoustic power may also be about 1.5mW/cm² to 60 mW/cm² or greater than 500mW/cm². In certain embodiments, the acoustic power may be about .5mW/cm², about 2mW/cm², about 3mW/cm², such as about 3.1mW/cm², about 4mW/cm² such as 4.2mW/cm², about 5mW/cm², about 15mW/cm², about 25mW/cm², about 30mW/cm² such as 30.1mW/cm², about 35mW/cm², about 50mW/cm², about 75mW/cm², or about 125mW/cm². In embodiments the acoustic power may range from about 2mW/cm² to 6mW/cm² or from about 25mW/cm² to 35mW/cm². One of skill in the art will appreciate that the relationship between the voltage input and the acoustic power output may vary for a particular transducer/ultrasonic frequency electrical signal generator combination versus a different transducer/ultrasonic frequency electrical signal generator combination.

In certain embodiments, the signal may be pulsed, while in some embodiments the signal may be continuous. In some embodiments the duty factor (pulse duration (sec) / pulse repetition period (sec) x 100) may be about 10%, about 20%, about 30%, about 40%, about about 50%, about 60%, about 70%, about 80%, about 90%, or about 100%. In some embodiments, the signal may be delivered continuously for about 30 seconds, about 1 minute, about 2 minutes, about 3 minutes, about 4 minutes, about 5 minutes, about 10 minutes, about 15 minutes, about 20 minutes, about 25 minutes, about 30 minutes, or greater than 30 minutes. Figures 13A-B depict calibration curves with accompanying data for two different therapeutic ultrasound apparatuses at 1.5MHz pulsed at 300 cycles/millisecond. The calibration curves show acoustic power output alongside the corresponding input voltage. As described above, one of skill in the art will understand that multiple factors within the therapeutic ultrasound apparatus will alter the output acoustic power.

By means of non-limiting examples, multiple experiments were conducted to determine the effectiveness of a therapeutic ultrasound signal, such as may be used in combination with the therapeutic ultrasound apparatuses of Figures 6-12. In brief, a modified fibroblast cell line with an impaired fibronectin Syndecan-4 signaling pathway (the healthy pathway indicated in Figure 5 above) was treated with ultrasound signals and the PAK phosphorylation was measured. A measurement of PAK phosphorylation is an indication of stimulation of the fibroblasts toward migration and healing activities, as shown above in Figure 5.

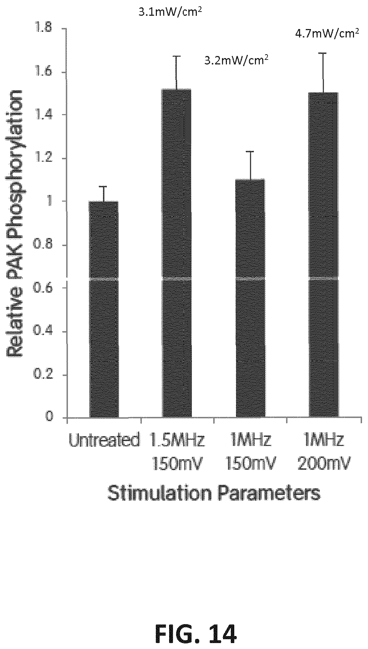

An example of one such non-limiting experiment is depicted in Figure 14. Cells treated with a frequency of 1MHz with acoustic power of 4.7mW/cm² and cells treated with a frequency of 1.5MHz and an acoustic power of 3.1mW/cm² showed an increase in PAK phosphorylation compared to untreated cells. Cells treated with 1MHz and 3.2mW/cm² showed a slight increase in PAK phosphorylation as compared to the untreated population, but the effect was minor and may be within the error range. Therefore, cells treated with a higher frequency had the same biochemical output as cells treated at a higher acoustic power but lower frequency, potentially indicating that 1.5MHz is more desirable than 1.0MHz.

Another example of a non-limiting experiment involving ultrasound and impaired fibroblasts is depicted in Figure 15 with a 1.5 MHz frequency signal, similar to the signal produced by the EXOGEN device produced by Bioventus. Here, the pulsed signal was pulsed at an acoustic power of 3.1mW/cm² and the continuous signal was delivered with an acoustic power of 2.6mW/cm², to simulate a relatively similar amount of vibrational energy delivery (acoustic power). However, even with a lower amount of vibrational energy delivery, the continuous signal produced a greater PAK phosphorylation, indicating that a continuous signal may provide improved wound healing over the pulsed signal.

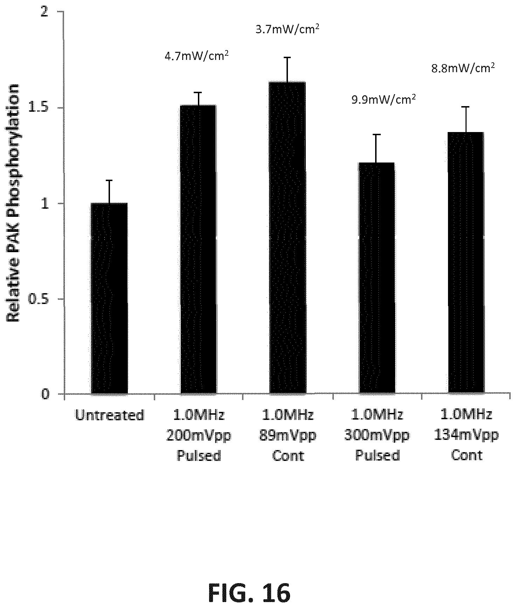

The results of another such non-limiting experiment involving the impaired fibroblasts described above is shown in Figure 16. Here, even with increasing power, at 1MHz, the continuous signal triggers a larger biochemical output as compared to the pulsed signal. However, the biochemical response for the continuous signal did not increase with increasing acoustic power at 1 MHz. This result may indicate a plateauing effect for increases in acoustic power for a continuous signal at 1MHz.

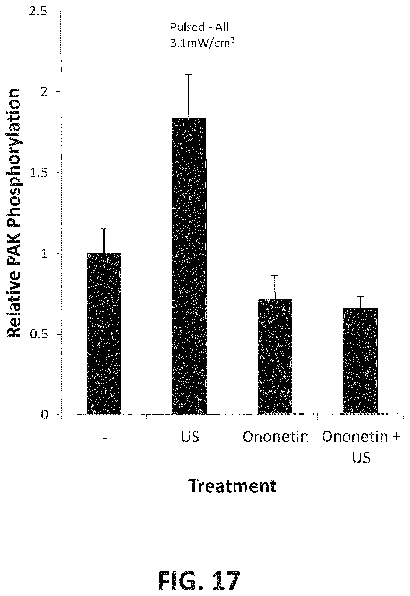

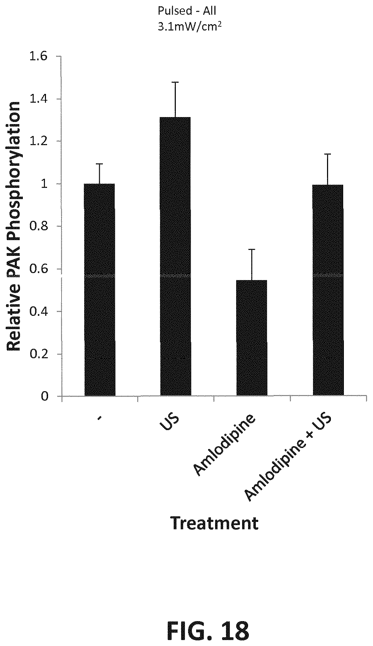

Figures 17-18 depict non-limiting experiments using a pulsed signal at 3.1mW/cm² involving the impaired fibroblasts described above. Here, the fibroblasts were blocked with various inhibitors and exposed to an ultrasound signal. As depicted in Figure 17, use of the calcium channel inhibitor Ononetin, which blocks trp-type calcium channels also blocked the ultrasound signal. In contrast, as shown in Figure 18, the ultrasound signal was not blocked through the use of the L-type calcium channel inhibitor amlodipine. These results suggest that the ultrasound mediated pathway involves a tryp-type calcium channel mediated pathway. These types of pathways are known to be sensitive to mechanical and temperature stimulation, such as may be delivered via vibrational energy.

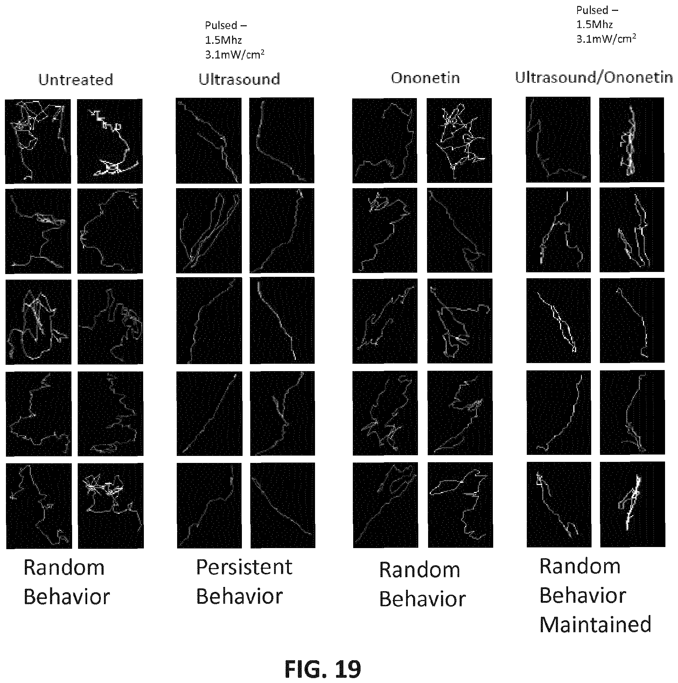

To further support the results of the experiment of Figure 17, a non-limiting migrational assay experiment was performed using a pulsed signal at 1.5MHz and 3.1mW/cm² to monitor the relative migration of the fibroblasts when subjected to ultrasound with or without Ononetin. Increased migration may be generally indicative of increased healing. As shown in Figure 19, when untreated, the fibroblasts show a random migration; however, when subjected to ultrasound, the fibroblasts display a controlled migration. However, this effect was lessened by treatment with the inhibitor Ononetin.

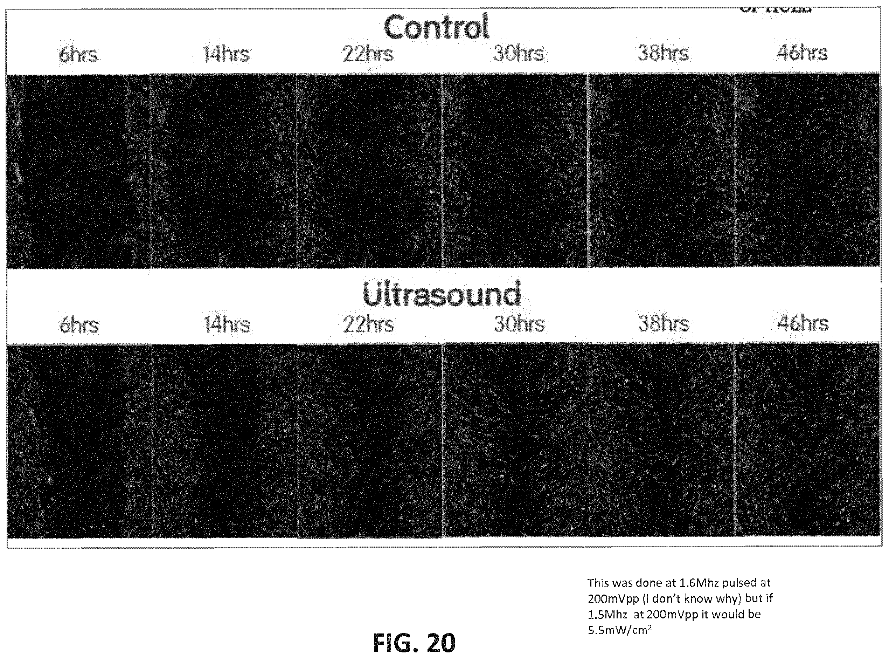

Figure 20 depicts a non-limiting experiment showing fibroblast migration across a gap over time while being subjected to an ultrasound signal of 1.6 MHz, and approximately 5.5mW/cm². Such an experiment is a good proxy for wound healing, as fibroblast migration across a gap may indicate a rate of healing across a wound. As shown in Figure 20, fibroblast migration across the gap is greatly increased when subjected to ultrasound over 46 hours, thereby indicating the potential for therapeutic ultrasound in wound healing.

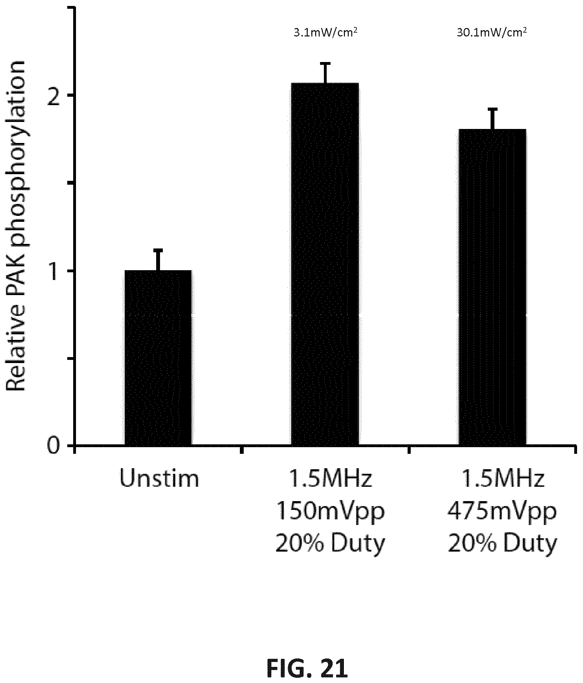

Figure 21 depicts another example of a non-limiting experiment, similar to the experiments of Figures 14 and 16. Here, for this particular set-up with a 20% duty cycle, 150mVpp corresponds to an acoustic power of approximately 3.1mW/cm², while 475mVpp corresponds to an acoustic power of approximately 30.1mW/cm². Here, with a 20% duty cycle, the relative PAK phosphorylation shows a slight decrease at 30.1m W/cm² as compared to 3.1mW/cm².

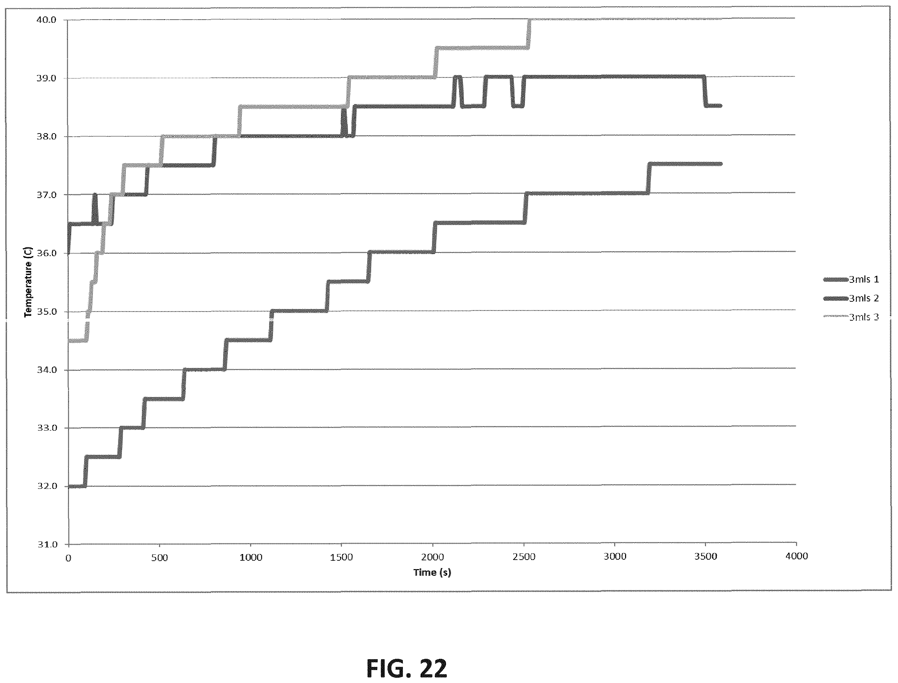

Figure 22 depicts another example of a non-limiting experiment to assess an increase in temperature with time during ultrasound treatment at 1.5MHz. Although the individual data replicates are highly variable, the average increase in temperature over the course of 20 minutes of treatment is 4.7 degrees Celsius. Therefore, one of skill in the art will understand that the temperature of an underlying substrate or tissue exposed to therapeutic ultrasound may increase over time during treatment.

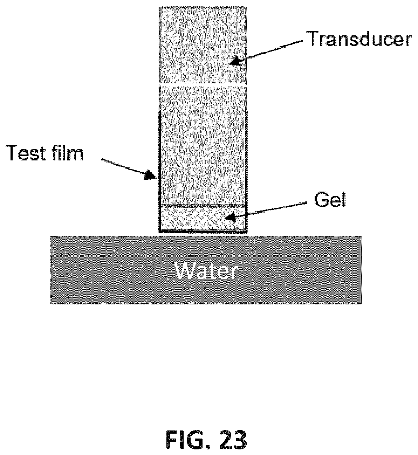

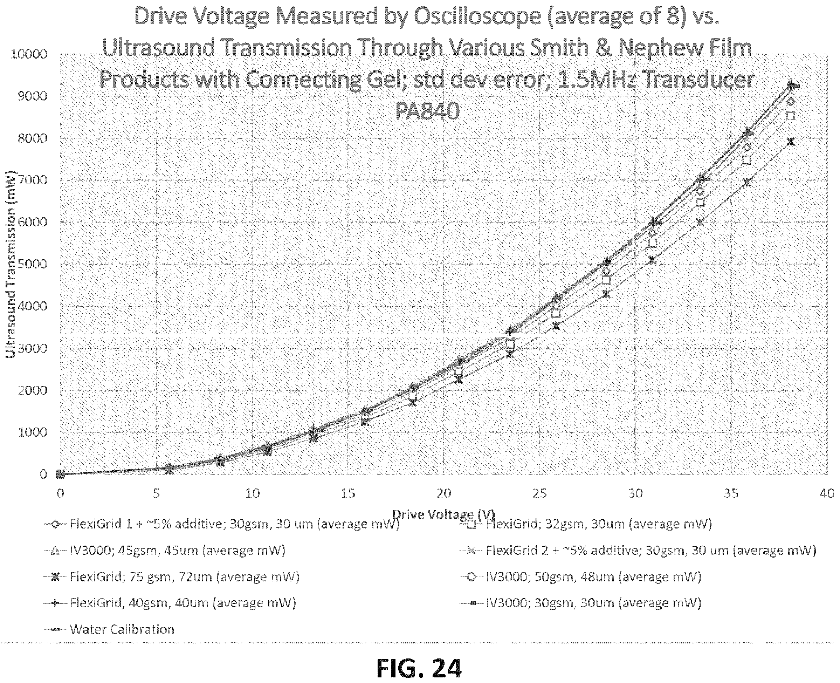

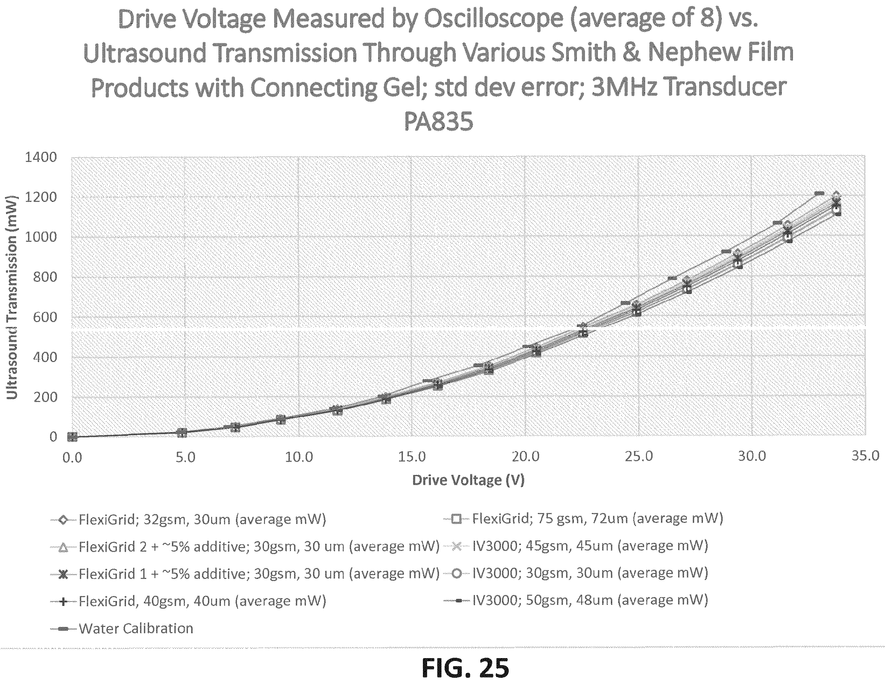

Figure 23 depicts an experimental setup for evaluating the ultrasound transmission of a layer of film. Here, a transducer is placed in direct contact with a transmissive gel, which is then wrapped in a layer of the test film. Ultrasound is then delivered through the gel and the test film into a water bath, where the ultrasound intensity is measures. Such an experiment allows one of skill in the art to assess the ultrasound transmission properties of the film. Table 1 below provides a listing of the various material films tested to generate the data provided in Figures 24 and 25. All films listed in Table 1 (below) are constructed from polyurethane.

**Table 1**

| **Sample type with batch number** | **Corresponding sample** |
|---|---|
| FlexiGrid; 80082574; 16500201 | FlexiGrid 1 + ^{~}5% additive; 30gsm, 30 um (average mW) |
| EU30; 80082578; 16500381 | FlexiGrid; 32gsm, 30um (average mW) |
| EU45; 80082345; 16500561SF | IV3000; 45gsm, 45um (average mW) |
| FlexiFix; 80082571; 16500684 | FlexiGrid 2 + ^{~}5% additive; 30gsm, 30 um (average mW) |
| EU75; 80083061-04; 16500610 | FlexiGrid; 75 gsm, 72um (average mW) |
| EU51 80083253 Roll 1 R/S | IV3000; 50gsm, 48um (average mW) |
| EU40 80082580; 16500291 | FlexiGrid, 40gsm, 40um (average mW) |
| EU33 pink AA top film 80082582; 16500537SF | IV3000; 30gsm, 30um (average mW) |

Figure 24 depicts a non-limiting experiment utilizing the films of Table 1 with the transducer and film setup depicted in Figure 25. Here, at 1.5MHz, all films showed an increase in ultrasound transmission power as drive voltage was increased. However, polyurethane film "IV3000," 45gsm, 45um showed the highest increase in ultrasound power with increasing drive voltage, while polyurethane film FlexiGrid, 40gsm, 40um (average mW) showed the smallest increase in power with increasing drive voltage.

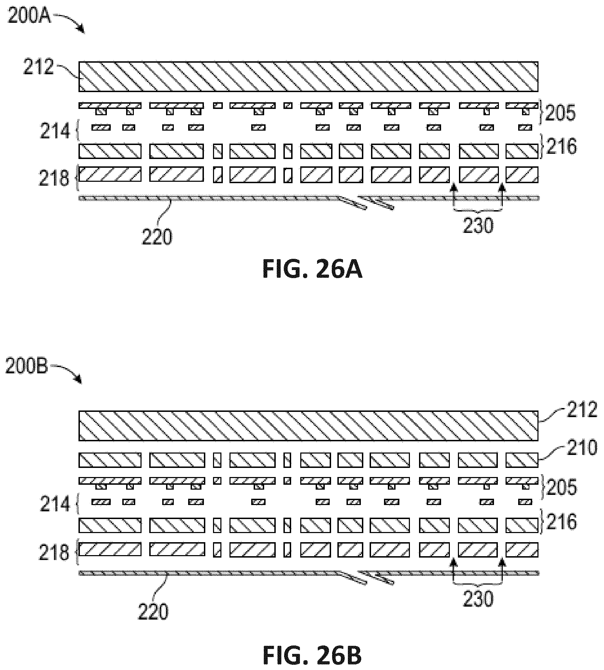

Figure 25 depicts a non-limiting experiment utilizing the films of Table 1 with the transducer and film setup depicted in Figure 25, similar to the experiment of Figure 26. Here, at 3.0MHz, all films showed an increase in ultrasound transmission power as drive voltage was increased. Polyurethane film FlexiGrid 32gsm, 30um (average mW) showed the highest increase in ultrasound power with increasing drive voltage, while polyurethane film IV3000; 50gsm, 48um (average mW) showed the lowest increase in ultrasound power with increasing drive voltage.

### Encapsulation and Stress Relief

In some cases, while it may be desirable for a substrate to be stretchable or substantially stretchable to better conform to or cover the wound, at least some of the electronic components or connections may not be stretchable or flexible. In such instances, undesirable or excessive localized strain or stress may be exerted on the one or more electronic components, such as on the supporting area or mountings of an electronic component, when the substrate is positioned in or over the wound. For example, such stress can be due to patient movement, changes in the shape or size of the wound (such as, due to its healing), or the like. Such stress may cause movement, dislodgment, or malfunction of the one or more electronic components or connections (for example, creation of an open circuit from a pin or another connector becoming disconnected). Alternatively or additionally, it may be desirable to maintain the position of one or more electronic components, such as one or more sensors or one or more ultrasonic transducers, in the same or substantially same location or region with respect to the wound (such as, in contact with the wound) so that measurements collected by the one or more electronic components accurately capture changes over time in the same or substantially same location or region of the wound. While the surface of the stretchable substrate may move when, for example, the patient moves, it may be desirable to maintain same or substantially same locations of one or more electronic components relative to the wound.

To address these problems, in some cases, non-stretchable or substantially non-stretchable coating (such coating can sometimes be referred to as "hard coat") can be applied to one or more electronic components (such as one or more ultrasonic transducers), one or more electronic connections, or the like. Hard coat can provide one or more of reinforcement or stress relief for one or more electronic components, one or more electronic connections, or the like. Hard coating can be formed from acrylated or modified urethane material. For example, hard coat can be one or more of Dymax 1901-M, Dymax 9001-E, Dymax 20351, Dymax 20558, Henkel Loctite 3211, or another suitable material. Hard coat can have viscosity from about 13.5 Pa·s to 50 Pa·s (13,500 cP to 50,000 cP) before being cured or from about 3.6 Pa·s to about 6.6 Pa·s (3,600 cP to about 6,600 cP) before being cured. In some cases, hard coat can have viscosity of no more than about 50 Pa·s (50,000 cP). Hard coat can have hardness from about D40 to about D65 and/or linear shrinkage of about 1.5-2.5%.

In some cases, another coating (or coatings) can be applied to encapsulate or coat one or more of the substrate or components supported by the substrate, such as the electronic connections or the electronic components. Coating can provide biocompatibility, shield or protect the electronics from coming into contact with fluids, provide padding for the electronic components to increase patient comfort, or the like. As used herein, biocompatible can mean being in compliance with one or more applicable standards, such as ISO 10993 or USP Class VI. Such coating cam be sometimes referred to as "conformal coat" or "soft coat." Soft coat can be stretchable or substantially stretchable. Soft coat can be hydrophobic or substantially hydrophobic.

Soft coat can be formed from one or more suitable polymers, adhesives, such as 1072-M adhesive (for example Dymax 1072-M), 1165-M adhesive (such as, NovaChem Optimax 8002-LV, Dymax 1165-M, or the like), 10901-M adhesive (for instance, Dymax 1901-M or 9001-E Dymax), parylene (such as, Parylene C), silicones, epoxies, urethanes, acrylated urethanes, acrylated urethane alternatives (such as, Henkel Loctite 3381), or other suitable biocompatible and substantially stretchable materials. Soft coat can be thin coating, for example, from about 80 microns or less up to several millimeters or more. Soft coat can have hardness lower than about A100, A80, A50 or lower. Soft coat can have elongation at break higher than about 100%, 200%, 300% or more. Soft coat can have viscosity of about 8.0-14.5 Pa·s (8,000-14,500 cP). In some cases, coating can have viscosity no less than about 3.0 Pa·s (3,000 cP). In some cases, coating can have viscosity less than about 3.0 Pa·s (3,000 cP).

Any of the hard or soft coats described herein can be applied by one or more of laminating, adhering, welding (for instance, ultrasonic welding), curing by one or more of light, UV, thermal (such as, heat), or the like. Any of the hard or soft coat described herein can be transparent or substantially transparent to facilitate optical sensing. Any of the coatings described herein can retain bond strength when subjected to sterilization, such as EtO sterilization. Any of the coatings described herein can be modified to fluoresce, such as under UV light.

FIGS. 26A-26B illustrate cross-sections of wound dressings that include ultrasonic transducer integrated substrates according to some embodiments. Dressing 200A shown in FIG. 26A can include an ultrasonic transducer integrated substrate 205 supporting a plurality of electronic components (shown as protruding from the substrate) and a plurality of electronic connections, as described herein. The dressing 200A can include hard coat 214, applied to one or more electronic components or connections. In some cases, hard coat can be applied to areas where electronic components are connected to electronic connections. This can reinforce these connections. In some cases, hard coat can be applied to each of the one or more of the electronic components or connections.

The dressing 200A can include soft coat 216, which can be applied to the entire wound facing side of the substrate. Soft coat 216 can be applied to an entire or substantially entire area of the wound facing side of the substrate to encapsulate the substrate, electronic components, and connections. In some cases, soft coat 216 can be applied to certain regions of the substrate, such as those regions supporting one or more of electronic components or connections.

The dressing 200A can include a wound contact layer 218. The wound contact layer 218 can include adhesive material configured to adhere the substrate to the wound, which can facilitate maintaining contact with the wound. The wound contact layer 218 can be formed from silicone. The silicone material can be low tac (or tack) silicone. The wound contact layer 218 can include silicone adhesive mounted on a film. In some cases, the wound contact layer 218 can be similar to the material used in Allevyn Life Non-Bordered dressing manufactured by Smith & Nephew.

The wound contact layer 218 can be applied to entire or substantially entire area of the wound facing side of the substrate. In some cases, the wound contact layer 218 can be applied to certain regions of the substrate, such as those regions supporting one or more of electronic components or connections.

The dressing 200A can include a protective layer 220 applied to the wound contact layer 218. The protective layer 220 can be made of paper, such as laminated paper. The protective layer 220 can protect the wound contact layer 218 prior to use and facilitate easy application for a user. The protective layer 218 can include a plurality (such as two) handles. The handles can be applied in a folded configuration, in which a slit separating the handles is covered by one of handles folded over the slit. In some cases, the protective layer 218 can be similar to the protective layer used in the Allevyn Life Non-Bordered dressing.

As illustrated, a wicking layer 212 can be positioned over an opposite, non-wound facing side of the substrate. The wicking layer 212 can facilitate passage of fluid through the layers below the wicking layer. For example, the wicking layer can transport (or "wick") fluid away from the lower layers, such as from the substrate, toward one or more upper layers positioned over the wicking layer 212. Such one or more upper layers can include one or more absorbent materials as described herein. In some cases, the wicking layer 212 is formed from foam, such as foam similar to that used in the Allevyn Life Non-Bordered dressing. The wicking layer can be extensible or substantially extensible.

As illustrated in the dressing 200B of FIG. 26B, additional layer of soft coat 210 can be positioned over the non-wound facing side of the substrate between the substrate and the wicking layer 212. For example, soft coat 210 can protect the non-wound facing side of the substrate from fluid if the substrate is formed from material that is not impermeable to fluid. In such case, soft coat 210 can be hydrophobic or substantially hydrophobic. Soft coat 210 can be made of same or different material than soft coat 218. Soft coat 210 can be perforated as illustrated and described. In some cases, soft coat can encapsulate the entire substrate, including both the wound facing and non-wound facing sides.

One of skill in the art will understand that the features of the embodiments of Figures 26A-26B, such as the hard and soft coats, may be combined with any of the embodiments described herein. For example, as described above, the hard and soft coats are compatible and useful for coating electrical components, including but not limited to electrical connectors and ultrasound transducers.

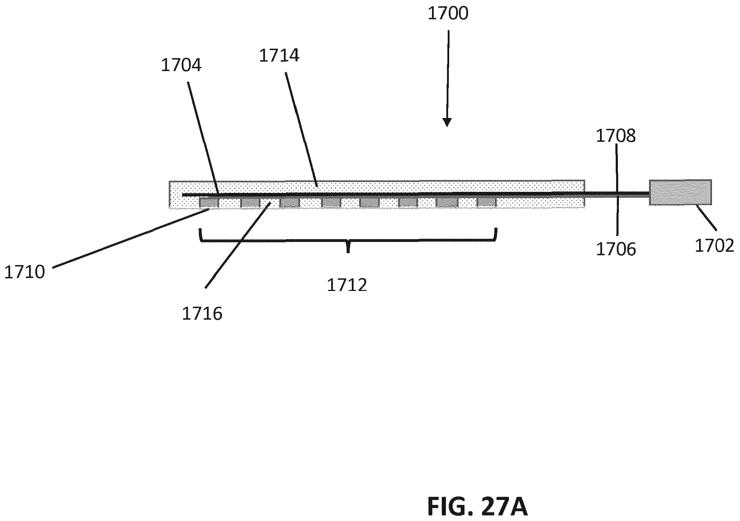

### Therapeutic Ultrasound Treatment Apparatuses of Figures 27A-28B

Figures 27A-27B depict an embodiment of a therapeutic ultrasound wound treatment apparatus 1700 for use in treating a wound, similar to the dressings described above in Figures 8A-11. Additionally, the features from the embodiments above, including but not limited to Figures 26A and 26B, may be combined with and/or incorporated into the embodiments of Figures 27A -28B. In certain embodiments, a suitable signal may be delivered to the dressing from an ultrasonic frequency electrical signal generator (not shown) via connector 1702 and via flexible integrated substrate 1704 including printed circuit track 1706 printed on a suitable material such as polyurethane film 1708 or any suitable material disclosed herein. One of skill in the art will understand that the signal may have any parameters such as timing, intensity, and frequency disclosed herein this section or elsewhere in the specification, particularly the parameters disclosed earlier in the specification with respect to Figures 13-17. The ultrasonic frequency electrical signal generator may be of any suitable type, for example, the EXOGEN generator manufactured by Bioventus.

In some embodiments, transducers 1710 in an array 1712 may be attached to the flexible integrated substrate 1704. The entire array may be encapsulated in a flexible protective coating 1714 (such as silicone, for example Silpuran 2111 & 2400-25 or any silicone disclosed herein) with vertical perforations 1716 between the ultrasonic transducers and the integrated substrate 1704 to allow for breathability and exudate removal. The apparatus 1700 of Figured 27A may function as a stand-alone apparatus positioned over a wound. In some embodiments, the therapeutic ultrasound wound treatment apparatus may function as a primary wound contact layer positioned over a wound and beneath a NPWT dressing such as the dressing 110 of Figure 3B.

Alternatively, the apparatus may be built into a wound dressing to act as the wound contact layer. For example, the apparatus 1700 may replace the wound contact layer 222 such as described in the dressing embodiment 110 of Figure 3B or any wound contact layer described herein. A layer of wicking material such as the wicking fabric of wicking layer 212 of Figure 3B or another suitable material may be placed over the top of the therapeutic ultrasound wound treatment apparatus to assist in removal and distribution of exudate into the dressing material.

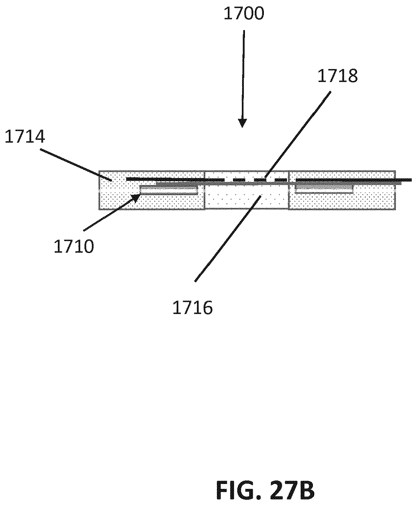

Figure 27B depicts a zoomed in version of an embodiment of the apparatus 1700 of Figure 27A, showing ultrasonic transducers 1710 separated by a perforation. Such a perforation may extend through openings 1718 of the integrated substrate (avoiding the transducers and electrical components), thereby allowing transmission of fluid such as wound exudate and/or gasses from the wound bed and through the wound treatment apparatus and optional overlying dressing. As described elsewhere in the specification, the perforations may be suitable for application of negative pressure wound therapy to the underlying wound bed.

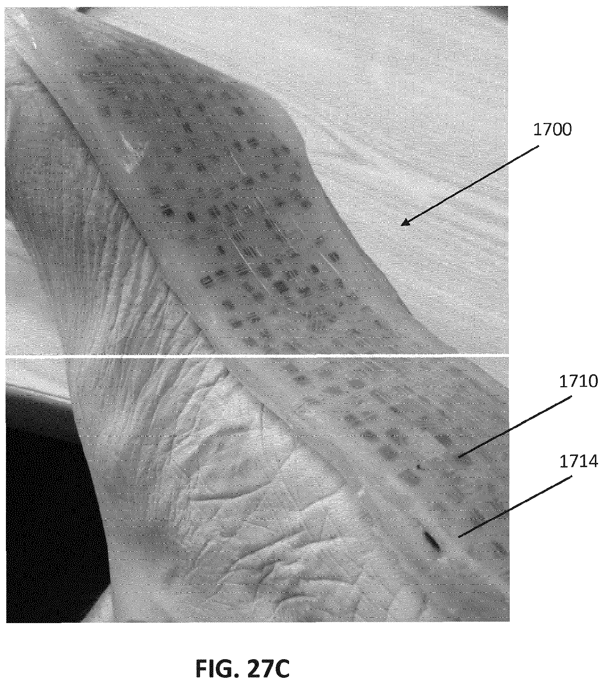

Figure 27C is a photograph of an embodiment of the ultrasound wound treatment apparatus 1700 depicted in Figures 27A-27B. Here, the ultrasound transducers 1710 are encased in a flexible protective coating 1714 (such as silicone), allowing the apparatus to flexibly conform to the bottom surface of a foot. Such an arrangement may be suitable to treat wounds on the bottom of the feet, including diabetic foot ulcers. However, one of skill in the art will understand that this example of a foot is non-limiting and such an ultrasound wound treatment apparatus may be applied to a wound on any surface of the body, such as the arm, leg, hand, abdomen, chest, back, head, or any suitable surface. One of skill in the art will further understand that such an ultrasound wound treatment apparatus 1700 may be smaller than the apparatus depicted in the figure and therefore suitable for placement on smaller areas of the body, such as a smaller portion of the foot such as the heel or other locations such as the hand. In certain embodiments, the wound treatment apparatus 1700 may be suitable for placement within a shoe and allow for treatment while the user is walking.

In embodiments, the wound treatment apparatus 1700 may be cuttable/trimmable to allow for modification of the apparatus for feet of different sizes, for example by cutting with a cutting device or tearing along pre-cut lines. For example, the integrated substrate may be functional such that upon removal of a section of the integrated substrate, the remaining ultrasonic transducers remain functional. In embodiments, pre-cuts may be applied to the wound treatment apparatus to allow for sections of the apparatus to be torn away to shape the apparatus for feet of varying size. Precuts may be applied to allow the apparatus to be sized to suitable men's or women's shoe sizes. Additionally, the wound treatment apparatus may be produced in left and right forms, with trimmable/removable sections to accommodate different foot sizes.

The wound treatment apparatus 1700 may include an indicator light, such as an LED light, that brightens when the apparatus is connected to an ultrasonic treatment generator and/or when ultrasound is being applied. Such an indicator light may change color or intensity according to the delivery of therapeutic ultrasound.

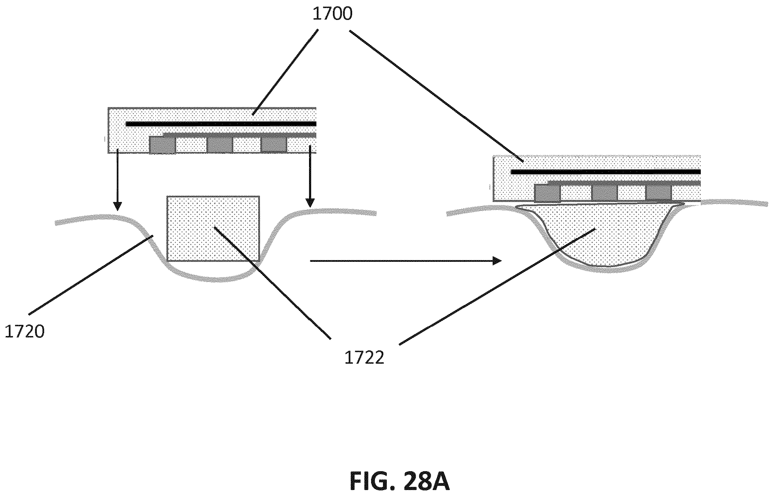

Figures 28A-28B depict embodiments of a therapeutic ultrasound wound treatment apparatus similar to the wound treatment apparatus of Figures 26A-27C. As described elsewhere in the specification, such as in relation to Figures 6-11, physical connectivity between the silicone outer surface of the wound treatment apparatus 1700 and the wound bed 1720 is often needed for sufficient ultrasound transmission. Such connectivity may be accomplished through the use of an ultrasound suitable gel or ultrasound suitable pillar and/or ultrasound suitable layer such as described elsewhere in the specification. In certain embodiments, an ultrasound transmission wound filler 1722 (such as a silicone) may be formed to the shape of the wound bed and thick enough to fill the cavity. The filler may be in the form of a thickened sheet or cube, and may be flexible enough to conform to the wound cavity after some amount of manipulation. As shown in Figure 28A, the ultrasound transmission wound filler 1722 may completely fill the wound cavity and be in contact with the wound treatment apparatus, thereby improving transmission of therapeutic ultrasound to the wound bed. One of skill in the art will understand that the wound filler may be made to any particular shape or size, such as a sheet, cube, or sphere; however, such a shape may be flexed and molded into the shape of the wound bed, either before placement into the wound or after. Further, the wound filler may be pre-shaped into the shape of the wound and then placed into the wound cavity, thereby requiring minimal manipulation.

In some embodiments, the ultrasound transmission wound filler 1722 may include perforations of suitable shape and size to accommodate the passage of air and wound exudate through the ultrasound transmission wound filler. As described elsewhere in the specification, such perforations may be suitable for the application ofNPWT to the underlying wound. The perforations in the ultrasound transmission wound filler and the perforations through the wound treatment apparatus may be matched such that the perforations align. In certain embodiments, the perforations in the ultrasound transmission wound filler and the perforations in the wound treatment apparatus may be of differing sizes to ensure overlap between the perforations in the wound filler and the perforations in the wound treatment apparatus.

As shown in Figure 28A, the wound treatment apparatus 1700 may be pressed against the ultrasound transmission wound filler 1722. Direct contact between the wound treatment apparatus 1700 and the ultrasound transmission wound filler 1722 may ensure that the therapeutic ultrasound is transmitted from the wound treatment apparatus through the ultrasound transmission wound filler and into the wound 1720. In embodiments, the wound treatment apparatus may be pressed down against the wound filler through the application of negative pressure, such as when the wound treatment apparatus 1700 functions as a wound contact layer for a wound dressing such as the dressing of Figure 3B. A wound cover connectable to negative pressure (not shown) may be placed over the wound treatment apparatus 1700, thereby allowing for the direct application of negative pressure wound therapy to the wound bed through the wound treatment apparatus and the ultrasound transmission wound filler while pressing the wound treatment apparatus against the wound filler through the application of vacuum force.

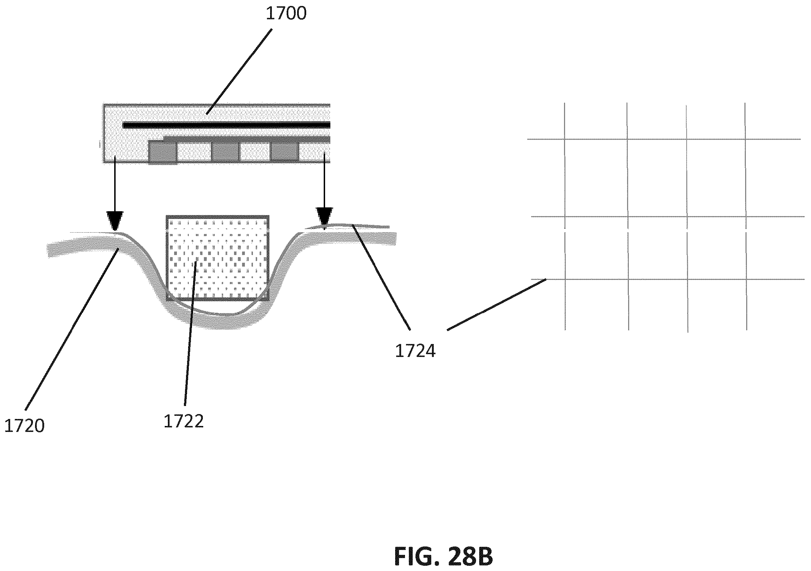

Figure 28B depicts the wound treatment apparatus 1700, ultrasound wound filler 1722, and wound bed 1720 of Figured 28A; however, here a layer of wicking material 1724 may be placed beneath the wound filler 1722. The wicking layer 1724 may be in the form of a fine net or other suitable arrangement, configured to channel wound exudate. Such a wicking layer may be large enough to wrap around and enfold the wound filler, and/or wrap around to enfold the wound filler and the wound treatment apparatus while the wound filler and the wound treatment apparatus are pressed together, thereby allowing for fluid wicking above and around the wound filler and wound treatment apparatus. Wicking of fluid from beneath the wound filler allows for the ultrasound transmission wound filler to be in direct contact with the wound bed, thereby allowing for proper transmission of therapeutic ultrasound. Such wicking layers will still wick wound exudate away in the absence of negative pressure, but may also be used in combination with negative pressure to enhance wound exudate removal.

In certain embodiments, wicking cores may also be placed within the perforations within the ultrasound transmission wound filler 1722 and/or the wound treatment apparatus 1700. Similar to the wicking layers, such wicking cores may wick away wound exudate from the wound bed with or without the application of negative pressure.

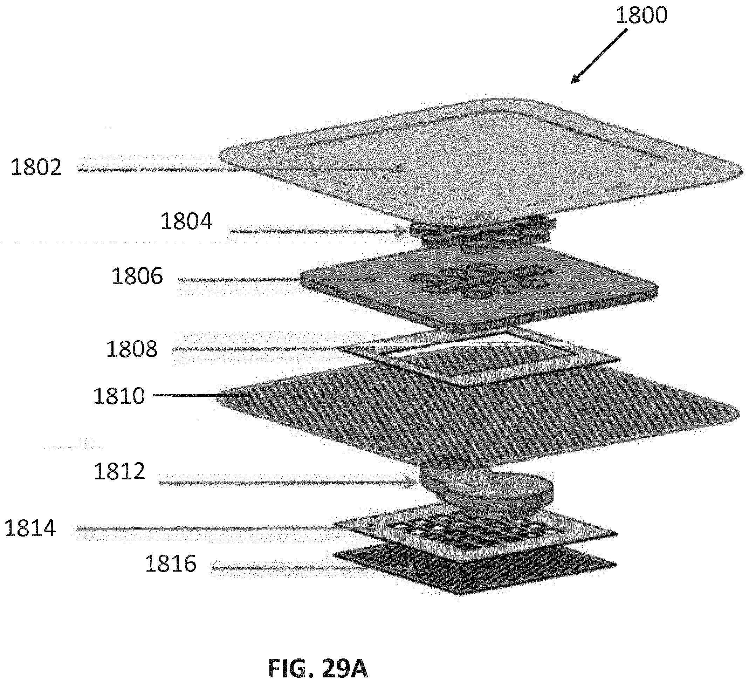

Figure 29A depicts an embodiment of a therapeutic ultrasound wound treatment apparatus 1800 configured to be placed over a tissue site such as a wound, similar to the therapeutic ultrasound wound treatment embodiments of Figures 6-11 and 26A-28B. One of skill in the art will understand that wires or other suitable connectors (not shown) may interface with the transducers to induce the generation of a therapeutic ultrasound signal, such as any suitable signal disclosed herein. Further, one of skill in the art will understand that although the various layers are described in a particular ordered layer here, other suitable sequences of layers may also contemplated, such as described elsewhere herein. Here, the wound treatment apparatus includes a backing layer 1802, which may be constructed from any of the materials used to construct a backing layer described herein such as transparent or opaque polyurethane film. Such a backing layer may overlie a transducer array 1804, similar to previous transducer arrays described here. Such a transducer array may be constructed according to any previous description of a transducer array described herein, for example such a transducer array may be encapsulated by a protective silicone coating and be configured to direct therapeutic ultrasound toward the wound. As explained above, such a transducer array may be connected to an ultrasonic frequency electrical signal generator (not shown) and/or power supply (not shown) and/or controller (not shown) as needed to generate therapeutic ultrasound. As shown in Figures 29A-29C, the transducer array may be constructed in a tree formation, with a central row of transducers and branches with additional transducers. In embodiments, the array may include between 1 and 20 transducers, such as 4-16 transducers, 6-14 transducers, 8-12 transducers, or about 10 transducers. The transducers may be held in a flexicarrier, constructed from any suitable material.

Continuing with Figure 29A, an absorbent layer 1806, similar to previous absorbent layers described herein may be positioned directly beneath, over, around, and/or surrounding the transducer array. For example, the absorbent layer 1806 may include a cutout for placement of the transducer array 1804. Such an absorbent layer 1806 may be constructed from any suitable absorbent material described herein, such as durafiber (as described herein), absorbent cellulose, superabsorbent particles or fibers, and/or viscose fibers. In certain embodiments, the absorbent layer may be a single layer thick, two layers thick, three layers thick, four layers think, or more than four layers thick. In embodiments, a first wicking layer 1808 may be positioned beneath the absorbent layer 1806, similar to other wicking layers described herein. One of skill in the art will understand that such a wicking layer may also be referred to as an acquisition distribution layer (ADL). Such a wicking layer may be constructed from any suitable material described herein, such as EVO80, and/or wicking fabrics, for example knitted or woven spacer fabrics (such as a knitted polyester 3D fabric, Baltex 7970^{®}, or Gehring 879^{®}) or a nonwoven fabric. In some embodiments, the wicking layer may be constructed from various materials, such as: a meltblown nonwoven treated with a hydrophilic nonwoven, a spunbond nonwoven treated with a hydrophilic nonwoven, a spunbond-meltblown-spunbond (SMS) nonwoven treated with a hydrophilic finish, a carded and needled bonded mixed fiber blend containing hydrophilic fibers in a range of dtex and cross sections (such as trilobal, Coolmax), a carded and oven bonded mixed fiber blend containing hydrophilic fibers in a range of dtex and cross sections (such as trilobal, Coolmax), or any suitable combinations thereof.

Continuing with Figure 29A, in embodiments, the first wicking layer 1808 may be in the shape of a rectangle, such as depicted in Figure 29A. However, the first wicking layer 1808 may also be in the shape of a circle, an oval, a square, a polygon, or any suitable shape. In certain embodiments, the first wicking layer may have the same shape of absorbent layer 1806, such that the first wicking layer 1808 may have a large surface area to wick fluid into the absorbent layer 1806. A wound contact layer 1810, similar to other wound contact layers described herein, may be positioned beneath the first wicking layer. Such a wound contact layer may interface directly with the wound, such as described elsewhere herein. In some embodiments, the wound contact layer may contain perforations such as described previously herein or the wound contact layer may contain no perforations, such that the wound contact layer is a continuous layer of silicone. By not containing perforations, the wound contact layer may advantageously improve therapeutic ultrasound transmission. The wound contact layer may be constructed from any suitable material described herein, such as silicone. In embodiments, this silicone wound contact layer may be the bottommost layer of the wound treatment apparatus 1800. However, as depicted in Figure 29A, additional components of the therapeutic ultrasound wound treatment apparatus may be positioned under the silicone wound contact layer 1810. For example, an ultrasound transmission wound filler layer 1812 may be placed in the form of sheets and/or plugs or any suitable shape beneath the wound contact layer 1810. Such transmission wound filler 1812 may be constructed from any suitable material described herein such as silicone, for example silicone rubbers such as Silpuran, for example Silpuran 2400/2. The transmission wound filler 1812 may be cut to the shape of the wound so as to provide a close fit and limit gaps whereby the therapeutic ultrasound signal may become attenuated and/or lost. The transmission wound filler 1812 may be constructed in layers, such as one layer, two layers, three layers, four layers, or more than four layers. One of skill in the art will understand that therapeutic ultrasound may be delivered through multiple dry or wet silicone layers. In embodiments, beneath the transmission wound filler layer may be a second wicking layer 1814, which may be constructed from any of the materials described in relation to the first wicking layer 1808. The second wicking layer may be in the form of a grid, a mesh, a net, or any suitable shape, similar to the wicking layer 1724 described above in relation to Figure 28B. Underlying the second wicking layer 1724 and interfacing directly with the wound may be positioned a second wound contact layer 1816. The second wound contact layer 1816 may be constructed from any suitable material described herein, such as silicone. As will be understood by one of skill in the art, the therapeutic ultrasound wound treatment apparatus 1800 may direct therapeutic ultrasound down through the various layers and into an underlying wound and/or intact tissue site and the surrounding tissue, such as skin.

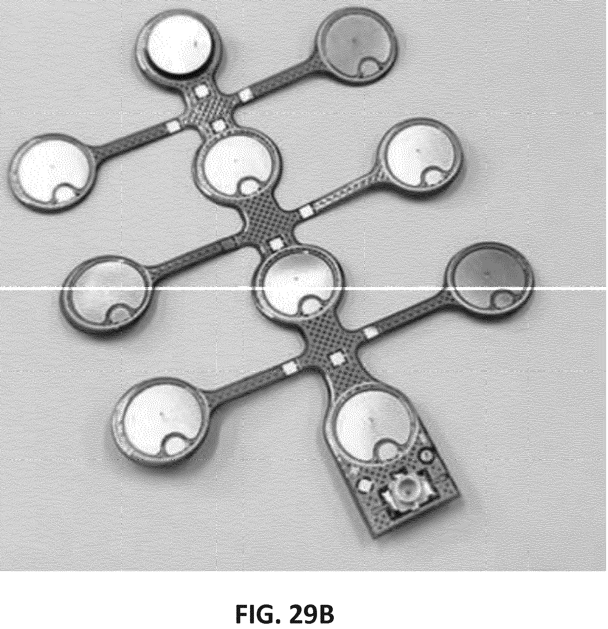

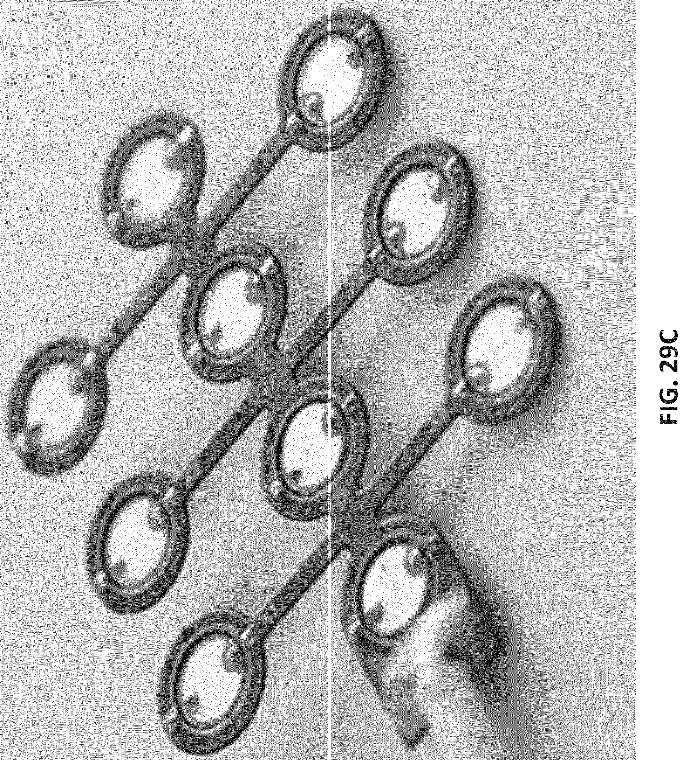

Figures 29B and 29C are photographs of embodiments of the transducer array 1804. As shown in Figure 29B, the transducers may be directly soldered into the array. In certain embodiments, the supporting structure of the array may be a printed circuit board (PCB) or any other suitable structure. As shown in Figure 29C, the transducers may be wire soldered into the array. By wire soldering directly into the array, durability and frequency control may be improved. For example, when directly soldering to a PCB, transducers may be susceptible to breakage and alter the frequency. As described above, such transducers may be arrayed into a tree arrangement, with branches for additional transducers. However, in certain embodiments, the transducers may be arrayed as a grid, a spiral, or any suitable arrangement.

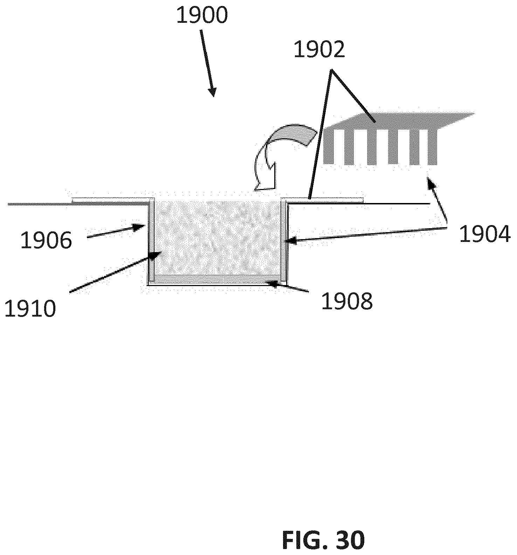

Figure 30 depicts an embodiment of a therapeutic ultrasound wound treatment system, suitable for use with any of the therapeutic wound treatment apparatuses and/or systems described herein. Here, a strip 1902 with wicking fingers 1904 may be placed into the edges of a wound bed 1906 over wound exudate 1908 and around an ultrasound transmission wound filler 1910, such as a silicone wound filler. The strip 1902 and wicking fingers 1904 may be constructed as a wound contact layer and wicking layer laminate, constructed from any suitable wound contact layer materials described herein and/or any suitable wicking layer materials described herein. In embodiments, the strip and wicking fingers may be constructed from only wicking materials, without the wound contact layer. Returning to the laminate embodiment, the wound contact layer side of the laminate may face and interface directly with the wound and periwound area, thereby allowing the fingers to extend downward into the wound bed and over the wound edges 1906 to extend into the wound exudate 1908. The wicking fingers 1904 may then serve to wick away wound exudate without interfering with the therapeutic ultrasound signal directed through the transmission wound filler. As described above, one of skill in the art will understand that other suitable therapeutic ultrasound dressings and systems may be layered over the transmission wound filler 1910or be incorporated with the transmission wound filler 1910 to allow for engagement of the wicking fingers 1904. Such wicking fingers may be suitable for any of the therapeutic ultrasound dressing or systems described herein, such as when it would be advantageous to remove wound exudate without disrupting the pathway of the therapeutic ultrasound into the wound bed. In certain embodiments, the laminate may be constructed in multiple layers, such as one layer, two layers, three layers, four layers, five layers or more than five layers.

As described herein, the transmission wound filler may be constructed to a suitable shape to fill a particular wound. Additionally, the transmission wound filler may be configured such that the filler extends above the wound cavity, such that it is higher than the surrounding skin surface, thereby potentially improving therapeutic ultrasound transmission. In some embodiments, with the wound cavity filled with the transmission wound filler, the wound exudate will be forced to the edges of the wound thereby drawn up by the wicking fingers.

Those skilled in the art will appreciate that in some embodiments, the actual steps taken in the processes illustrated or disclosed may differ from those shown in the figures. Depending on the embodiment, certain of the steps described above may be removed, others may be added. For example, the actual steps or order of steps taken in the disclosed processes may differ from those shown in the figure. Depending on the embodiment, certain of the steps described above may be removed, others may be added. For instance, the various components illustrated in the figures may be implemented as software or firmware on a processor, controller, ASIC, FPGA, or dedicated hardware. Hardware components, such as processors, ASICs, FPGAs, and the like, can include logic circuitry. Furthermore, the features and attributes of the specific embodiments disclosed above may be combined in different ways to form additional embodiments, all of which fall within the scope of the present disclosure.

## Claims

1. A therapeutic ultrasound wound treatment apparatus (1000) comprising:
a wound contact layer;
a delivery layer (1016) positioned over the wound contact layer, the delivery layer comprising a porous portion and a transmission portion (1018), the transmission portion comprising a transmission material configured to transmit vibrational energy;
an ultrasonic transducer (1014), the ultrasonic transducer positioned over the transmission portion, the ultrasonic transducer configured to deliver vibrational energy at a therapeutic frequency;
an absorbent layer (1012) positioned over the delivery layer, the absorbent layer configured to absorb wound exudate; and
a cover layer (1010) overlying the absorbent layer; and
wherein the transmission portion extends through an entire thickness of the delivery layer.

2. The apparatus of Claim 1, wherein the transmission portion comprises silicone gel.

3. The apparatus of Claim 1, wherein the ultrasonic transducer comprises a piezoelectric transducer.

4. The apparatus of Claim 1, further comprising a fluid connector attached to the cover layer, the fluid connector in communication with a space under the cover layer.

5. The apparatus of Claim 4, wherein the fluid connector is configured to be in fluid communication with a source of negative pressure.

6. The apparatus of Claim 1, wherein there are a plurality of ultrasonic transducers and a plurality of transmission portions, each ultrasonic transducer positioned over a transmission portion and configured to deliver vibrational energy at a therapeutic frequency

7. The apparatus of Claim 6, wherein the plurality of ultrasonic transducers comprises five ultrasonic transducers, the five ultrasonic transducers arranged in a cross pattern.

8. A therapeutic ultrasound treatment system, comprising:
the therapeutic ultrasound wound treatment apparatus of any one of Claims 1-7; and
an ultrasound transmission wound filler configured to be positioned beneath the wound treatment apparatus.

9. The system of Claim 8, wherein the ultrasound transmission wound filler comprises a plurality of perforations.

10. The system of Claim 8 or Claim 9, further comprising a wicking layer, the wicking layer configured to be positioned beneath the ultrasound transmission wound filler.

11. The system of Claim 10, wherein the wicking layer comprises a net.

12. The system of Claim 10 or Claim 11, wherein the wicking layer is configured to fold over and cover the ultrasound transmission wound filler.

13. The system of Claim 10 or Claim 11, further comprising wicking fingers, the wicking fingers configured to be positioned around the perimeter of the ultrasound transmission wound filler.

14. The apparatus of Claims 1 to 7 or the system of Claims 8 to 13, wherein the vibrational energy is controlled by a controller, the controller configured to apply vibrational energy at a frequency of 1.5 MHz.

## Patentansprüche

1. Therapeutische Ultraschall-Wundbehandlungsvorrichtung (1000), umfassend:
eine Wundkontaktschicht;
eine Abgabeschicht (1016), die über der Wundkontaktschicht angeordnet ist, wobei die Abgabeschicht einen porösen Abschnitt und einen Übertragungsabschnitt (1018) umfasst, wobei der Übertragungsabschnitt ein Übertragungsmaterial umfasst, das dafür ausgelegt ist, Vibrationsenergie zu übertragen;
einen Ultraschallwandler (1014), wobei der Ultraschallwandler über dem Übertragungsabschnitt angeordnet ist, wobei der Ultraschallwandler dafür ausgelegt ist, Vibrationsenergie mit einer therapeutischen Frequenz abzugeben;
eine absorbierende Schicht (1012), die über der Abgabeschicht angeordnet ist, wobei die absorbierende Schicht dafür ausgelegt ist, Wundexsudat aufzusaugen; und
eine Deckschicht (1010), die über der absorbierenden Schicht liegt; und
wobei sich der Übertragungsabschnitt durch eine gesamte Dicke der Abgabeschicht erstreckt.

2. Vorrichtung nach Anspruch 1, wobei der Übertragungsabschnitt Silikongel umfasst.

3. Vorrichtung nach Anspruch 1, wobei der Ultraschallwandler einen piezoelektrischen Wandler umfasst.

4. Vorrichtung nach Anspruch 1, die ferner einen an der Deckschicht angebrachten Fluidanschluss umfasst, wobei der Fluidanschluss mit einem Raum unter der Deckschicht in Kommunikationsverbindung steht.

5. Vorrichtung nach Anspruch 4, wobei der Fluidanschluss dafür ausgelegt ist, mit einer Unterdruckquelle in Fluidverbindung zu stehen.

6. Vorrichtung nach Anspruch 1, wobei es eine Mehrzahl von Ultraschallwandlern und eine Mehrzahl von Übertragungsabschnitten gibt, wobei jeder Ultraschallwandler über einem Übertragungsabschnitt angeordnet ist und dafür ausgelegt ist, Vibrationsenergie mit einer therapeutischen Frequenz abzugeben.

7. Vorrichtung nach Anspruch 6, wobei die Mehrzahl von Ultraschallwandlern fünf Ultraschallwandler umfasst, wobei die fünf Ultraschallwandler in einem Kreuzmuster angeordnet sind.

8. Therapeutisches Ultraschallbehandlungssystem, umfassend:
die therapeutische Ultraschall-Wundbehandlungsvorrichtung nach einem der Ansprüche 1-7; und
einen Ultraschallübertragungs-Wundfüller, der dafür ausgelegt ist, unter der Wundbehandlungsvorrichtung angeordnet zu werden.

9. System nach Anspruch 8, wobei der Ultraschallübertragungs-Wundfüller eine Mehrzahl von Perforationen aufweist.

10. System nach Anspruch 8 oder Anspruch 9, ferner eine Dochtschicht umfassend, wobei die Dochtschicht dafür ausgelegt ist, unter dem Ultraschallübertragungs-Wundfüller angeordnet zu werden.

11. System nach Anspruch 10, wobei die Dochtschicht ein Netz umfasst.

12. System nach Anspruch 10 oder Anspruch 11, wobei die Dochtschicht dafür ausgelegt ist, den Ultraschallübertragungs-Wundfüller zu überlappen und zu bedecken.

13. System nach Anspruch 10 oder Anspruch 11, ferner Dochtstreifen umfassend, wobei die Dochtstreifen dafür ausgelegt sind, unter dem Ultraschallübertragungs-Wundfüller angeordnet zu werden.

14. Vorrichtung nach Anspruch 1 bis 7 oder System nach Anspruch 8 bis 13, wobei die Vibrationsenergie durch eine Reglereinheit geregelt wird, wobei die Reglereinheit dafür ausgelegt ist, Vibrationsenergie mit einer Frequenz von 1,5 MHz anzuwenden.

## Revendications

1. Appareil thérapeutique de traitement de plaies par ultrasons (1000) comprenant :
une couche de contact avec la plaie ;
une couche d'administration (1016) placée au-dessus de la couche de contact avec la plaie, la couche d'administration comprenant une partie poreuse et une partie de transmission (1018), la partie de transmission comprenant un matériau de transmission configuré pour transmettre une énergie vibratoire ;
un transducteur à ultrasons (1014), le transducteur à ultrasons étant positionné au-dessus de la partie de transmission, le transducteur à ultrasons tant configuré pour administrer une énergie vibratoire à une fréquence thérapeutique ;
une couche absorbante (1012) placée au-dessus de la couche d'administration, la couche absorbante étant configurée pour absorber l'exsudat de la plaie ; et
une couche de recouvrement (1010) recouvrant la couche absorbante ; et
dans lequel la partie de transmission s'étend à travers toute l'épaisseur de la couche d'administration.

2. Appareil selon la revendication 1, dans lequel la partie de transmission comprend un gel de silicone.

3. Appareil selon la revendication 1, dans lequel le transducteur à ultrasons comprend un transducteur piézoélectrique.

4. Appareil selon la revendication 1, comprenant en outre un raccord de fluide fixé à la couche de recouvrement, le raccord de fluide étant en communication avec un espace sous la couche de recouvrement.

5. Appareil selon la revendication 4, dans lequel le raccord de fluide est configuré pour être en communication fluidique avec une source de pression négative.

6. Appareil selon la revendication 1, dans lequel il y a une pluralité de transducteurs à ultrasons et une pluralité de parties de transmission, chaque transducteur à ultrasons étant positionné sur une partie de transmission et configuré pour administrer une énergie vibratoire à une fréquence thérapeutique.

7. Appareil selon la revendication 6, dans lequel la pluralité de transducteurs à ultrasons comprend cinq transducteurs à ultrasons, les cinq transducteurs à ultrasons étant agencés selon un motif en croix.

8. Système de traitement thérapeutique à ultrasons, comprenant :
l'appareil thérapeutique de traitement de plaies par ultrasons selon l'une quelconque des revendications 1 à 7 ; et
un produit de remplissage de plaie à transmission d'ultrasons configuré pour être positionné sous l'appareil de traitement de plaies.

9. Système selon la revendication 8, dans lequel le produit de remplissage de plaie à transmission d'ultrasons comprend une pluralité de perforations.

10. Système selon la revendication 8 ou la revendication 9, comprenant en outre une couche à effet de mèche, la couche à effet de mèche étant configurée pour être positionnée au-dessous du produit de remplissage de plaie à transmission d'ultrasons.

11. Système selon la revendication 10, dans lequel la couche à effet de mèche comprend un filet.

12. Système selon la revendication 10 ou la revendication 11, dans lequel la couche à effet de mèche est configurée pour se replier et recouvrir le produit de remplissage de plaie à transmission d'ultrasons.

13. Système selon la revendication 10 ou la revendication 11, comprenant en outre des doigts à effet de mèche, les doigts à effet de mèche étant configurés pour être positionnés autour du périmètre du produit de remplissage de plaie à transmission d'ultrasons.

14. Appareil selon les revendications 1 à 7 ou système selon les revendications 8 à 13, dans lequel l'énergie vibratoire est commandée par un dispositif de commande, le dispositif de commande étant configuré pour appliquer une énergie vibratoire à une fréquence de 1,5 MHz.
